# EUROPEAN PATENT APPLICATION

(11) **EP 4 803 113 A1**
(43) Date of publication of application: **09.09.2026**
(21) Application number: 24884844.2
(22) Date of filing: 30.10.2024
(51) Int. Cl.: A61M 1/06

(54) **BREAST PUMP ASSEMBLY, BREAST PUMP, BREAST PUMP APPARATUS, AND DUST COVER**

(30) Priority: 31.10.2023 CN 202322953512 U; 31.10.2023 CN 202322946621 U; 31.10.2023 CN 202322945261 U; 31.10.2023 CN 202322948544 U; 31.10.2023 CN 202322946846 U; 31.10.2023 CN 202322946626 U; 31.10.2023 CN 202322950064 U; 31.10.2023 CN 202322948572 U
(71) Applicant: Shenzhen Lute Jiacheng Supply Chain Management Co., Ltd., Shenzhen, Guangdong 518000 (CN)
(72) Inventor: CHEN, Wanyuan, Shenzhen, Guangdong 518000 (CN); LI, Tianlei, Shenzhen, Guangdong 518000 (CN); XIE, Guangbao, Shenzhen, Guangdong 518000 (CN)
(74) Representative: Santarelli
(86) International application number: PCT/CN2024/128630
(87) International publication number: WO 2025/092844

(57) **Abstract**

The present disclosure provides a breast pump assembly, a breast pump, a breast pump device, and a dust cover. The breast pump assembly includes a container body, a sealing cover, a negative pressure member, and a milk guide member, where the container body and the sealing cover enclose a milk storage chamber; the negative pressure member is connected to the sealing cover; the negative pressure member has a negative pressure chamber; the negative pressure chamber is configured to communicate with a negative pressure hole of the breast pump; the milk guide member is connected to the sealing cover; the milk guide member has a milk guide channel; the milk guide channel communicates with the negative pressure chamber; the milk guide channel is at least configured to guide milk from a breast; the negative pressure member, the milk guide member, and the sealing cover are integrally formed; and one of the milk guide channel and the negative pressure chamber communicates with the milk storage chamber. The breast pump assembly provided by the present disclosure has a smaller number of components, lowering the assembly difficulty, and improving the assembly efficiency. In addition, the present disclosure further prevents the problem of poor airtightness due to assembly errors of the negative pressure member and the milk guide member.

## Description

### Cross-reference to Related Applications

The present disclosure claims the priority to Chinese Patent Application No. 202322946846.1, Chinese Patent Application No. 202322948544.8, Chinese Patent Application No. 202322946626.9, Chinese Patent Application No. 202322950064.5, Chinese Patent Application No. 202322953512.7, Chinese Patent Application No. 202322948572X, Chinese Patent Application No. 202322945261.8, and Chinese Patent Application No. 202322946621.6, filed on October 31, 2023, all of which are incorporated herein by reference in their entirety.

### Technical Field

The present disclosure relates to the technical field of maternal and infant products, and in particular, to a breast pump assembly, a breast pump, a breast pump device, and a dust cover.

### Background of the Invention

Breast pumps are designed for postpartum women. When breastfeeding is not practical, lactating women can use the breast pump to express milk from the breast for storage and subsequent use. This prevents the waste of breast milk, and facilitates the work and lives of the lactating women. Therefore, the breast pumps have gained widespread popularity in the market.

Typically, a breast pump includes a milk guide member having a milk guide channel and a negative pressure member having a negative pressure chamber. Through the negative pressure chamber, a negative pressure is transferred to the milk guide channel, thereby expressing the milk from the breast. In the related art, the milk guide member and the negative pressure member are detachably assembled together. This results in an excessive number of components of the breast pump, increasing assembly difficulty, and causing more assembly errors and potential airtightness hazards.

### Summary of the Invention

A first objective of the present disclosure is to provide a breast pump assembly, to solve the technical problem that the existing breast pump has an excessive number of components to cause large assembly difficulty and potential airtightness hazards.

To achieve the above objective, the present disclosure provides the following solutions: A breast pump assembly is configured for a breast pump, and includes: a container body and a sealing cover, where the container body and the sealing cover enclose a milk storage chamber; a negative pressure member, where the negative pressure member is connected to the sealing cover, the negative pressure member has a negative pressure chamber, and the negative pressure chamber is configured to communicate with a negative pressure hole of the breast pump; and a milk guide member, where the milk guide member is connected to the sealing cover, the milk guide member has a milk guide channel, the milk guide channel communicates with the negative pressure chamber, and the milk guide channel is at least configured to guide milk from a breast; the negative pressure member, the milk guide member, and the sealing cover are integrally formed, and one of the milk guide channel and the negative pressure chamber communicates with the milk storage chamber.

As an implementation, both the milk guide member and the negative pressure member are connected to a side of the sealing cover facing the milk storage chamber.

As an implementation, a first perforation and a second perforation are formed in the sealing cover; a central axis of the first perforation is parallel to a central axis of the second perforation; the negative pressure member is connected to the first perforation; the first perforation is configured to communicate the negative pressure chamber with the negative pressure hole; the milk guide member is connected to the second perforation; and the second perforation communicates with the milk guide channel, and is configured to allow the milk to pass through and flow to the milk guide channel.

As an implementation, the central axis of the first perforation is coaxial with a central axis of the negative pressure chamber; and the central axis of the second perforation is coaxial with a central axis of the milk guide channel.

As an implementation, the first perforation is closely adjacent to the second perforation; and/or when the breast pump is placed against the breast, the first perforation is located under the second perforation.

As an implementation, when the breast pump is placed against the breast, the first perforation is located directly under the second perforation, and the negative pressure chamber communicates with the milk storage chamber.

As an implementation, the container body is provided with a first mounting groove; and the sealing cover is accommodated in the first mounting groove, so as to enclose the milk storage chamber with the container body.

A second objective of the present disclosure is to provide a breast pump, including: a breast shield, where the breast shield is provided with a breast accommodating chamber; a main unit, where the main unit is provided with a negative pressure hole configured to transfer a negative pressure; and the breast pump assembly, where the milk guide channel communicates with the breast accommodating chamber, and the negative pressure chamber communicates with the negative pressure hole.

As an implementation, the first perforation is formed in the sealing cover; the negative pressure member is connected to the first perforation and located on the side of the sealing cover facing the milk storage chamber; the breast pump further includes an elastic diaphragm; the elastic diaphragm includes a diaphragm body and a turnup connected to the diaphragm body; a circumferential direction of the main unit around the negative pressure hole and/or a circumferential direction of the sealing cover around the first perforation is provided with a second mounting groove; the turnup is disposed in the second mounting groove; and the diaphragm body is accommodated in the negative pressure chamber.

As an implementation, the negative pressure member further has a first through hole and a second through hole; the first through hole is configured to communicate the negative pressure chamber with the milk guide channel; the second through hole is configured to communicate the negative pressure chamber with the milk storage chamber; when the breast pump is placed against the breast, the negative pressure chamber is located under the milk guide channel; the elastic diaphragm has a contracted state and an expanded state; and when the elastic diaphragm is in the expanded state, at least a portion of the elastic diaphragm corresponding to the first through hole is spaced apart from a wall of the negative pressure chamber.

According to the breast pump assembly and the breast pump provided by the present disclosure, both the negative pressure member and the milk guide member are connected to the sealing cover, and the negative pressure member, the milk guide member, and the sealing cover are integrally formed. That is, the negative pressure member, the milk guide member, and the sealing cover are designed into a whole. This reduces the number of components of the breast pump assembly, thereby lowering the assembly difficulty of the breast pump assembly, and improving the assembly efficiency. In addition, the operation of assembling the negative pressure member and the milk guide member on the sealing cover is omitted, thereby preventing the problem of poor airtightness due to assembly errors of the negative pressure member and the milk guide member.

A third objective of the present disclosure is to provide a breast pump, to solve the technical problem that the user cannot quickly and effectively get a problem of the breast pump in use.

To achieve the above objective, the present disclosure provides the following solutions: A breast pump includes: a milk guide member, where the milk guide member is made of an optically transparent material and has a milk guide channel, and the milk guide channel is at least configured to guide milk from a breast; a negative pressure member, where the negative pressure member has a negative pressure chamber, and the negative pressure chamber at least communicates with the milk guide channel; and a main unit, where the main unit is provided with a negative pressure hole, and the negative pressure hole communicates with the negative pressure chamber; and when the breast pump is placed against the breast, the negative pressure member is located under the milk guide member, and the main unit is located at a position other than on the milk guide member.

As an implementation, at least a portion of the main unit is disposed on a side of the negative pressure member away from the milk guide member.

As an implementation, the negative pressure member is made of the optically transparent material.

As an implementation, the breast pump further includes a milk storage container; both the milk guide member and the negative pressure member are connected to an inner side of the milk storage container, and the main unit is disposed on an outer side of the milk storage container; and when the breast pump is placed against the breast, at least a portion of the milk storage container located above the milk guide member is made of the optically transparent material.

As an implementation, the milk storage container is entirely made of the optically transparent material.

As an implementation, the milk storage container includes a first container wall and a second container wall; the first container wall and the second container wall enclose a milk storage chamber; both the negative pressure member and the milk guide member are connected to a side of the second container wall facing the milk storage chamber; when the breast pump is placed against the breast, the first container wall is located above the milk guide member; and transparency of the first container wall is greater than or equal to transparency of the second container wall.

As an implementation, the breast pump further includes a breast shield; the breast shield includes a breast attachment portion and a channel portion connected to the breast attachment portion; the breast attachment portion is configured to be adsorbed to the breast; the channel portion is provided with a nipple channel; and at least a portion of the channel portion extends into the milk guide member, such that the nipple channel communicates with the milk guide channel.

As an implementation, the channel portion is made of the optically transparent material.

As an implementation, the channel portion is in an interference fit with the milk guide member; or an outer surface of the channel portion is smooth; or the breast shield is detachably connected to the milk guide member.

As an implementation, an end of the channel portion away from the breast attachment portion and an inner surface of the milk guide member define a step, so as to prevent backflow of the milk.

According to the breast pump provided by the present disclosure, the milk guide member having the milk guide channel is provided, and configured to deliver the milk from the breast, and the milk guide member is made of the optically transparent material, such that the user can directly view a condition in the milk guide channel. Since the negative pressure member is disposed under the milk guide member, and the main unit is disposed at the position other than on the milk guide member, when the breast pump is used, the user views the breast pump from the position above the breast pump, and neither the negative pressure member nor the main unit shields the milk guide member. Thus, the user can view a flowing condition of the milk in the milk guide channel in real time. In case of an excessively fast or slow flow rate, an operation mode can be adjusted at any time, so as to slow down or speed up flowing of the milk. Therefore, when the breast pump provided by the present disclosure is used, the user can quickly and effectively get the problem of the breast pump to immediately adjust the operation mode, greatly improving the use effect of the breast pump.

A fourth objective of the present disclosure is to provide a breast pump assembly, to solve the technical problem of the excessively large size of the breast pump.

To achieve the above objective, the present disclosure provides the following solutions: A breast pump assembly is configured for a breast pump, and includes: a negative pressure member, where the negative pressure member has a negative pressure chamber, and the negative pressure chamber is configured to communicate with a negative pressure hole of the breast pump; and a milk guide member, where the milk guide member has a milk guide channel, the milk guide channel communicates with the negative pressure chamber, and the milk guide channel is at least configured to guide milk from a breast; and the milk guide member and the negative pressure member are arranged side by side in the breast pump.

As an implementation, when the breast pump is placed against the breast, the negative pressure member is located under the milk guide member.

As an implementation, the milk guide member and the negative pressure member share a first side wall; two opposite sides of the first side wall face the negative pressure chamber and the milk guide channel, respectively; and a first through hole configured to communicate the negative pressure chamber with the milk guide channel is formed in the first side wall.

As an implementation, when the breast pump is placed against the breast, the first through hole is located at a lowest position of the milk guide member.

As an implementation, the negative pressure member further includes a second side wall and a first bottom wall; and the first side wall, the second side wall, and the first bottom wall enclose the negative pressure chamber; and
a second through hole configured to communicate with negative pressure chamber is formed in the second side wall; and when the breast pump is placed against the breast, the second through hole is located under the first through hole.

As an implementation, when the breast pump is placed against the breast, the second through hole is located at a lowest position of the negative pressure member.

As an implementation, when the breast pump is placed against the breast, the second through hole directly faces the first through hole along a vertical direction; or, the second through hole is formed in an end of the second side wall close to the first bottom wall.

As an implementation, a length of the milk guide member is the same as a length of the negative pressure member; and/or, an opening of the negative pressure chamber for communicating with the negative pressure hole and an opening of the milk guide channel for receiving the milk are arranged side by side and have a same orientation.

As an implementation, the breast pump assembly includes a container body and a sealing cover; the container body and the sealing cover enclose a milk storage chamber; and the milk guide member and the negative pressure member are connected side by side to a same surface of the sealing cover and extend toward one side of the milk storage chamber.

A fifth objective of the present disclosure is to provide a breast pump, including: a breast shield, where a breast accommodating chamber is formed in the breast shield; a main unit, where the main unit is provided with a negative pressure hole configured to transfer a negative pressure; and the breast pump assembly, where the milk guide channel communicates with the breast accommodating chamber, and the negative pressure chamber communicates with the negative pressure hole.

According to the breast pump assembly and the breast pump provided by the present disclosure, the milk guide member and the negative pressure member are arranged side by side in the breast pump, i.e., the milk guide member and the negative pressure member have a same extension direction, so the breast pump only needs to provide an enough space in a direction same as the extension direction of the milk guide member and the negative pressure member to accommodate the milk guide member and the negative pressure member. In contrast to the arrangement where the milk guide member and the negative pressure member are crossed, and the breast pump provides enough accommodating spaces in two different extension directions for the milk guide member and the negative pressure member, the present disclosure effectively reduces the occupied space of the breast pump assembly. Therefore, the breast pump assembly provided by this embodiment has a small occupied space, thereby reducing the overall size of the breast pump.

A sixth objective of the present disclosure is to provide a main unit of a breast pump, to solve the technical problems of unreasonable structural design and large occupied space of the existing main unit.

To achieve the above objective, the present disclosure provides the following solutions: A breast pump includes: a main unit housing, where the main unit housing includes a first shell and a second shell extending relative to the first shell in a bending manner, so as to form a step on the main unit housing, and an accommodating chamber is formed in the main unit housing; a milk pumping assembly, where the milk pumping assembly is at least partially located on the step; and a pumping power device that is disposed in the accommodating chamber, where the pumping power device is configured to generate a negative pressure.

As an implementation, the milk pumping assembly includes a milk collection unit and a breast shield; the milk collection unit is disposed on one side of the first shell; the second shell is disposed between the breast shield and the milk collection unit; and at least one of the first shell and the second shell has a cavity, so as to form the accommodating chamber.

As an implementation, the first shell has a first cavity; the second shell has a second cavity; the accommodating chamber includes the first cavity and the second cavity; and at least a portion of the pumping power device is accommodated in the first cavity.

As an implementation, the pumping power device includes an air pump, a solenoid valve, a battery, and a circuit board; the air pump, the solenoid valve, and the battery are accommodated in the first cavity; and the circuit board is accommodated in the second cavity.

As an implementation, the air pump, the battery, and the solenoid valve are arranged side by side; and the battery is located between the air pump and the solenoid valve.

As an implementation, a negative pressure hole is formed in a side of the second shell provided with the first shell; and the negative pressure hole communicates with the second cavity.

As an implementation, the breast pump further includes a key unit; and the key unit is disposed on a circumferential side, other than an end close to the first shell, of the second shell.

As an implementation, the second shell is further provided with a through hole; the through hole extends from a side of the second shell away from the first shell to a side of the second shell close to the first shell; and the through hole is configured to allow the breast shield to penetrate through.

As an implementation, the milk collection unit is supported on the first shell; and/or, the main unit housing is L-shaped; and an L-shaped step is formed on the main unit housing.

As an implementation, the milk collection unit includes a milk storage container and a breast pump assembly extending into the milk storage container; and the pumping power device is configured to provide the negative pressure for the breast pump assembly.

According to the breast pump provided by the present disclosure, the second shell extends relative to the first shell in the bending manner, the step is formed on the main unit housing, and the milk pumping assembly is at least partially located on the step, such that the main unit housing and the milk pumping assembly are closely assembled together. This arrangement is reasonable, and greatly reduces the occupied space of the main unit housing and the overall size of the breast pump, thereby making the breast pump convenient to carry and use, and enhancing the user experience.

A seventh objective of the present disclosure is to provide a breast pump, to solve the technical problems of the large size and weight of the existing breast pump.

To achieve the above objective, the present disclosure provides the following solutions: A breast pump includes: a main unit, where the main unit is configured to provide a negative pressure; and a milk storage container, where the milk storage container includes a support wall and an enclosure wall, the support wall and the enclosure wall enclose a milk storage chamber, and the support wall is configured to support milk flowing to the milk storage chamber; and at least a portion of the main unit is attached to a side of the support wall away from the milk storage chamber.

As an implementation, the main unit includes a first bottom shell, a first top shell, and an air pump; the first bottom shell and the first top shell enclose a first accommodating chamber; the air pump is accommodated in the first accommodating chamber; and the first top shell is attached to the side of the support wall away from the milk storage chamber.

As an implementation, a projected contour of the first top shell along a direction toward the support wall falls within a contour of the support wall.

As an implementation, a thickness of the first top shell is less than or equal to a thickness of the first bottom shell.

As an implementation, the first top shell includes at least two panels; any two adjacent ones of the panels are disposed at an included angle; and the included angle is less than 180°.

As an implementation, each of the panels is non-planar; and/or, two adjacent ones of the panels are in smooth transition.

As an implementation, the at least two panels define a limiting recess; and the limiting recess is configured to limit the milk storage container.

As an implementation, the air pump corresponds to a bottom wall of the limiting recess.

As an implementation, one of the first top shell and the support wall is provided with an anti-disengagement snap groove, while the other is provided with an anti-disengagement snap protrusion; and the anti-disengagement snap protrusion is snap-fitted into the anti-disengagement snap groove.

As an implementation, the main unit further includes a solenoid valve; and the air pump and the solenoid valve are accommodated side by side in the first accommodating chamber.

According to the breast pump provided by the present disclosure, with the milk storage container, expressed milk can flow to the milk storage chamber, and is supported on the support wall. At least a portion of the main unit is attached to the side of the support wall away from the milk storage chamber. When the milk is supported on the support wall, since the temperature of the milk is lower than the temperature of the main unit, the milk can absorb heat of the main unit, thereby achieving a heat dissipation effect for the main unit. In contrast to the heat dissipation method using heat sinks, this arrangement omits the heat sinks, greatly reduces the overall size and weight of the device, and further enhances the user experience.

In view of shortages in the prior art, the present disclosure provides a breast pump device and a dust cover having a charging function, to balance the milk capacity and the battery endurance of the breast pump, and ensure the portability and user experience of the breast pump.

To achieve the above objective, the present disclosure adopts the following technical solutions: A dust cover having a charging function is configured to cooperate with a breast pump for use, and includes: a cover body, where an accommodating chamber is formed in the cover body, and the cover body is configured to be mounted on the breast pump, so as to shield an opening of the breast pump; and a charging assembly that is at least partially disposed in the accommodating chamber, and configured to charge the breast pump when the cover body is mounted on the breast pump.

As an implementation, the charging assembly includes: an interface module that is configured to match with the breast pump for electrical connection when the cover body is mounted on the breast pump; and a power source that is electrically connected to the interface module, where the power source is a battery or an external power source.

As an implementation, the interface module includes a first conductive terminal; the breast pump includes a second conductive terminal; and the first conductive terminal is configured to contact the second conductive terminal for electrical conduction when the cover body is mounted on the breast pump.

As an implementation, the first conductive terminal is a conductive pin protruding from the cover body; a recess is formed in the breast pump; the second conductive terminal is embedded into the recess; and when the cover body is mounted on the breast pump, the conductive pin can extend into the recess to contact the second conductive terminal.

As an implementation, the interface module includes a first inductive coil; the breast pump includes a second inductive coil; and when the cover body is mounted on the breast pump, electric energy can be transmitted between the first inductive coil and the second inductive coil.

As an implementation, a surface of the cover body facing the opening of the breast pump is provided with a protruding conical portion; and the first inductive coil is disposed in the accommodating chamber, and located at a position corresponding to the conical portion.

As an implementation, the cover body is L-shaped; the cover body includes a body portion and a base portion connected to the body portion in a bending manner; the body portion is configured to shield the opening of the breast pump; and the base portion is configured to support the breast pump.

As an implementation, the first conductive terminal is disposed on a top surface of the base portion.

As an implementation, the interface module is disposed on a first surface of the cover body; and the first surface is adjacent to the surface of the cover body facing the opening of the breast pump.

Another objective of the present disclosure is to provide a breast pump device, including a breast pump and the dust cover having a charging function.

After the dust cover provided by the present disclosure is mounted on the breast pump, it can shield the opening of the breast pump to prevent entry of dust, bacteria and the like into the breast pump and avoid the contamination and deterioration to the milk in the breast pump. Meanwhile, the breast pump can be charged as required without increasing the size of the breast pump, which balances the milk capacity and the battery endurance of the breast pump, and ensures the portability and user experience of the breast pump.

In view of shortages in the prior art, the present disclosure provides a breast pump device and a dust cover, to prevent the cold breast pump from directly contacting a human breast, thereby enhancing the user experience.

To achieve the above objective, the present disclosure adopts the following technical solutions: A dust cover is configured to cooperate with a breast pump for use, and includes: a cover body, where the cover body is configured to be mounted on the breast pump, so as to shield a milk collection opening of the breast pump, a mounting space is formed in the cover body, and an air outlet communicating with the mounting space is formed in the cover body; and a heating assembly, where the heating assembly is disposed in the mounting space and configured to heat air in the mounting space, and the air heated by the heating assembly is discharged from the air outlet to preheat the breast pump.

As an implementation, the cover body further includes an air inlet communicating with the mounting space; and the dust cover further includes: a convection fan that is disposed in the mounting space and configured to draw outside air into the mounting space from the air inlet, and blow the air heated by the heating assembly out from the air outlet.

As an implementation, the heating assembly includes a first heating element; and the first heating element is disposed between the air inlet and the convection fan.

As an implementation, the heating assembly includes a second heating element; and the second heating element is disposed between the convection fan and the air outlet.

As an implementation, the heating assembly includes at least one heating element; and the at least one heating element is a heating sheet, and/or the at least one heating element is a heating wire, and/or the at least one heating element is a heating mesh.

As an implementation, the dust cover further includes a cylindrical guide shell mounted in the mounting space; the convection fan is disposed in the guide shell; and two ends of the guide shell face the air inlet and the air outlet, respectively.

As an implementation, a side of the cover body facing the milk collection opening is convexly provided with a conical guide portion; and the air outlet is formed in an end surface and/or an outer circumferential surface of a free end of the guide portion.

As an implementation, the air inlet is formed in a side of the cover body facing away from the milk collection opening and/or an outer circumferential surface of the cover body.

Another objective of the present disclosure is to provide a breast pump device, including a breast pump and the dust cover.

As an implementation, when the dust cover is configured to be mounted on the milk collection opening of the breast pump, a gap is formed between the dust cover and the milk collection opening; and heated air discharged from the air outlet can be discharged from at least one of a milk pouring opening of the breast pump and the gap.

The dust cover provided by the present disclosure can be mounted on the milk collection opening of the breast pump to protect the breast pump, preventing entry of bacteria, dust and the like into the breast pump. Moreover, due to the heating assembly disposed in the dust cover, when the dust cover is mounted on the breast pump, hot air generated in the dust cover can preheat the breast pump, thereby preventing the cold breast pump from directly contacting the human breast to enhance the user experience.

In view of shortages in the prior art, the present disclosure provides a breast pump device and a dust cover. The present disclosure can prevent the milk collection channel from growing bacteria to contaminate the milk in the breast pump, thereby safeguarding the health of infants.

To achieve the above objective, the present disclosure adopts the following technical solutions: A dust cover is configured to cooperate with a breast pump for use, and includes: a cover body, where the cover body is configured to be mounted on the breast pump, so as to shield a milk collection opening of the breast pump, a mounting space is formed in the cover body, and the cover body has a light exiting side facing the milk collection opening; and a disinfection assembly that is at least partially disposed in the mounting space and configured to emit ultraviolet light through the light exiting side to disinfect the milk collection opening of the breast pump.

As an implementation, the light exiting side of the cover body is provided with a conical protrusive portion and an accommodating portion around the protrusive portion; the accommodating portion is configured to allow the breast pump to be partially embedded into when the protrusive portion enters the milk collection opening; the protrusive portion includes an annular conical surface; and the conical surface is light-transmissive, or, a plurality of light-transmitting windows are formed in the conical surface.

As an implementation, the disinfection assembly includes a lamp panel and a plurality of ultraviolet light sources that are disposed in the mounting space and electrically connected; the lamp panel is fixed on the cover body; and the ultraviolet light sources are disposed on the lamp panel at intervals along a circumferential direction of the cover body and face the light exiting side.

As an implementation, the disinfection assembly includes a plurality of light guide posts; and each of the light guide posts includes one end facing the ultraviolet light sources, and another end extending to the conical surface, and inclining toward a center of the conical surface, so as to guide the ultraviolet light emitted by the ultraviolet light sources to exit through the conical surface.

As an implementation, the disinfection assembly includes a light guide ring; the light guide post is connected to the light guide ring; and a surface of the light guide ring facing away from the light guide post is attached to a surface of the lamp panel provided with the ultraviolet light sources.

As an implementation, the disinfection assembly includes a light homogenizing sheet; and the light homogenizing sheet is attached to an inner surface of the protrusive portion and directly faces the conical surface.

As an implementation, the conical surface is a reflective surface.

As an implementation, the milk collection opening includes a breast accommodating chamber and a nipple channel; the disinfection assembly includes at least one ultraviolet light source; and a light exiting direction of the ultraviolet light source is configured to incline toward a center of the nipple channel.

Another objective of the present disclosure is to provide a breast pump device, including a breast pump and the dust cover, where the dust cover is detachably connected to the breast pump.

As an implementation, the breast pump includes a flange and a milk bowl disposed on the flange; the milk bowl is at least partially made of a transparent material; a breast shield having the milk collection opening is formed on the flange; the breast shield is made of an opaque material; and the ultraviolet light emitted by the disinfection assembly is irradiated onto the milk collection opening.

The dust cover provided by the present disclosure can shield the milk collection opening of the breast pump, preventing entry of bacteria, dust and the like into the breast pump. Meanwhile, since the dust cover further includes the disinfection assembly, when the dust cover shields the breast pump, the milk collection opening can be disinfected and sterilized, preventing the milk collection opening from breeding bacteria to contaminate the milk in the breast pump, thereby safeguarding the health of infants.

### Brief Description of the Drawings

FIG. 1 is a schematic structural view of a breast pump assembly from a viewing angle according to an embodiment of the present disclosure;
FIG. 2 is a rear view of a breast pump assembly according to an embodiment of the present disclosure;
FIG. 3 is a sectional view along a line A-A in FIG. 2;
FIG. 4 is an exploded view of a breast pump assembly according to an embodiment of the present disclosure;
FIG. 5 is a schematic view of an assembly structure of a sealing cover, a negative pressure member, and a milk guide member according to an embodiment of the present disclosure;
FIG. 6 is a schematic structural view of a container body according to an embodiment of the present disclosure;
FIG. 7 is a schematic structural view of a breast pump from a viewing angle according to an embodiment of the present disclosure;
FIG. 8 is a rear view of a breast pump according to an embodiment of the present disclosure;
FIG. 9 is a sectional view along a line B-B in FIG. 8;
FIG. 10 is an exploded view of a breast pump according to an embodiment of the present disclosure;
FIG. 11 is a schematic structural view of an elastic diaphragm according to an embodiment of the present disclosure;
FIG. 12 is a schematic structural view of a breast pump from a viewing angle according to an embodiment of the present disclosure;
FIG. 13 is a rear view of a breast pump according to an embodiment of the present disclosure;
FIG. 14 is a sectional view along a line C-C in FIG. 13;
FIG. 15 is a schematic view of an assembly structure of a milk guide member, a negative pressure member, and a milk storage container according to an embodiment of the present disclosure;
FIG. 16 is a schematic structural view of a breast shield according to an embodiment of the present disclosure;
FIG. 17 is a schematic structural view of a breast pump assembly from a viewing angle according to an embodiment of the present disclosure;
FIG. 18 is a rear view of a breast pump assembly according to an embodiment of the present disclosure;
FIG. 19 is a sectional view along a line D-D in FIG. 18;
FIG. 20 is an exploded view of a breast pump assembly according to an embodiment of the present disclosure;
FIG. 21 is a schematic view of an assembly structure of a sealing cover, a negative pressure member, and a milk guide member according to an embodiment of the present disclosure;
FIG. 22 is a schematic structural view of a breast pump from a viewing angle according to an embodiment of the present disclosure;
FIG. 23 is a rear view of a breast pump according to an embodiment of the present disclosure;
FIG. 24 is a sectional view along a line E-E in FIG. 23;
FIG. 25 is an exploded view of a breast pump according to an embodiment of the present disclosure;
FIG. 26 is a schematic structural view of a breast pump from a viewing angle according to an embodiment of the present disclosure;
FIG. 27 is a rear view of FIG. 26;
FIG. 28 is a sectional view along a line F-F in FIG. 27;
FIG. 29 is an exploded view of FIG. 26;
FIG. 30 is a schematic view of an assembly structure of a main unit housing and a pumping power device from a viewing angle according to an embodiment of the present disclosure;
FIG. 31 is a right view of FIG. 30;
FIG. 32 is a front view of FIG. 30;
FIG. 33 is a sectional view along a line G-G in FIG. 32;
FIG. 34 is an exploded view of FIG. 30;
FIG. 35 is a schematic structural view of a breast pump from a viewing angle according to an embodiment of the present disclosure;
FIG. 36 is a rear view of the breast pump shown in FIG. 35;
FIG. 37 is a schematic structural view of a milk storage container according to an embodiment of the present disclosure;
FIG. 38 is a schematic structural view of a main unit from a viewing angle according to an embodiment of the present disclosure;
FIG. 39 is a front view of the main unit shown in FIG. 38;
FIG. 40 is a sectional view along a line H-H in FIG. 39;
FIG. 41 is a schematic structural view of the main unit shown in FIG. 38 from another viewing angle;
FIG. 42 is a sectional view of a panel according to an embodiment of the present disclosure;
FIG. 43 is a sectional view of another panel according to an embodiment of the present disclosure;
FIG. 44 is a sectional view of still another panel according to an embodiment of the present disclosure;
FIG. 45 is an exploded view of another main unit according to an embodiment of the present disclosure;
FIG. 46 is a schematic structural view of a breast pump device according to an embodiment of the present disclosure;
FIG. 47 is a sectional view of a breast pump according to an embodiment of the present disclosure;
FIG. 48 is a schematic exploded view of a breast pump device according to an embodiment of the present disclosure;
FIG. 49 is another schematic exploded view of a breast pump device according to an embodiment of the present disclosure;
FIG. 50 is a schematic structural view of a breast pump device according to an embodiment of the present disclosure;
FIG. 51 is a schematic sectional view of a dust cover according to an embodiment of the present disclosure;
FIG. 52 is a schematic exploded view of a breast pump device according to an embodiment of the present disclosure;
FIG. 53 is a schematic sectional view of a breast pump device according to an embodiment of the present disclosure;
FIG. 54 is a schematic structural view of a breast pump device according to an embodiment of the present disclosure;
FIG. 55 is a sectional view of a breast pump according to an embodiment of the present disclosure;
FIG. 56 is a schematic exploded view of a breast pump device according to an embodiment of the present disclosure; and
FIG. 57 is another schematic exploded view of a breast pump device according to an embodiment of the present disclosure.

### Detailed Description of Embodiments

The technical solutions in the embodiments of the present application are clearly and completely described below with reference to the accompanying drawings in the embodiments of the present application. Apparently, the described embodiments are merely some rather than all of the embodiments of the present application. All other embodiments obtained by those of ordinary skill in the art based on the embodiments of the present application without creative efforts should fall within the protection scope of the present application.

It should be noted that all the directional indications (such as upper, lower, left, right, front, back, etc.) in the examples of the present application are merely used to explain a relative position relationship, motion situations, and the like of the components in a specific gesture. If the specific gesture changes, the directivity indication also changes accordingly.

It should be noted that when a component is ''fixed'' or ''provided'' on the other component, the component may be ''fixed'' to or ''provided'' on the other component directly or via an intermediate component. When a component is ''connected'' to the other component, the component may be ''connected'' to the other component directly or via an intermediate component.

Moreover, the terms such as "first", "second", and the like described in the present application are used herein only for the purpose of description and are not intended to indicate or imply relative importance, or implicitly indicate the number of the indicated technical features. Therefore, features defined by "first" and "second" may explicitly or implicitly include at least one of the features. Furthermore, the technical solutions between the various embodiments may be combined with each other, but must be on the basis that the combination thereof can be implemented by a person of ordinary skill in the art. In case of a contradiction with the combination of the technical solutions or a failure to implement the combination, it should be considered that the combination of the technical solutions does not exist, and is not within the protection scope of the present disclosure.

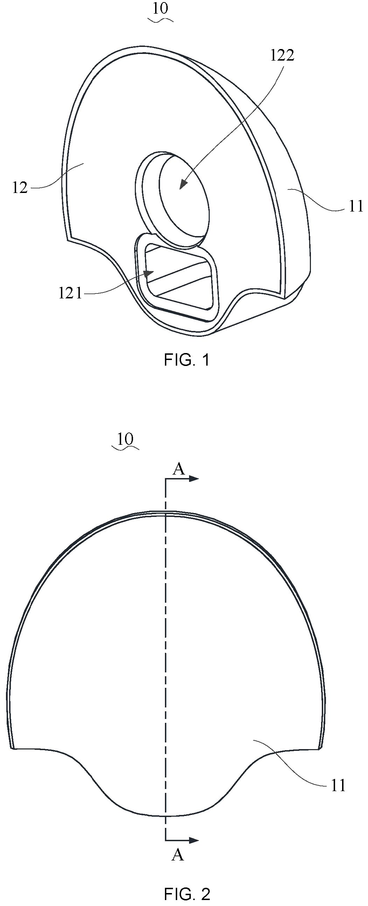

As shown in FIGS. 1-3 and FIG. 7, an embodiment of the present disclosure provides a breast pump assembly 10 for a breast pump 100, including a container body 11 and a sealing cover 12. The container body 11 and the sealing cover 12 enclose a milk storage chamber 111. In this way, the milk can be temporarily stored in the milk storage chamber 111 well.

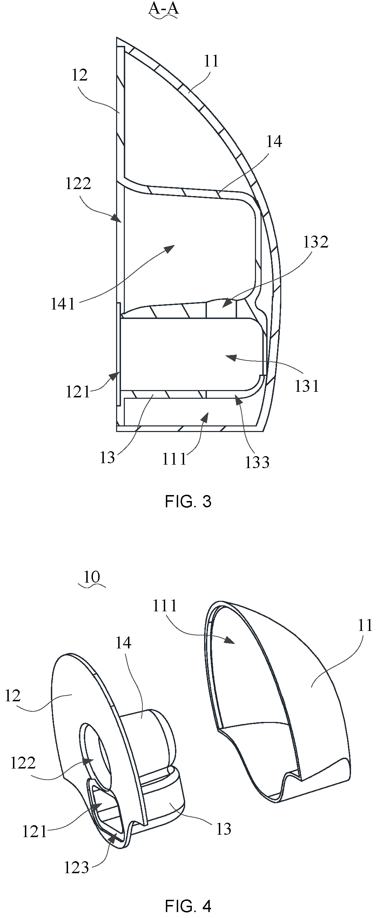

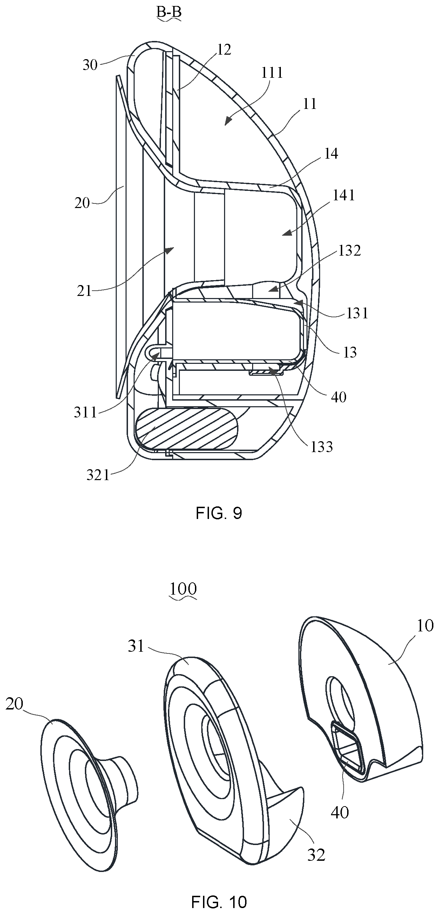

As an implementation, referring to FIGS. 3-5, FIG. 8, and FIG. 9, the breast pump assembly 10 further includes a negative pressure member 13 and a milk guide member 14. Both the negative pressure member 13 and the milk guide member 14 are connected to the sealing cover 12. The negative pressure member 13 has a negative pressure chamber 131. The negative pressure chamber 131 is configured to communicate with a negative pressure hole 311 of the breast pump 100. The milk guide member 14 has a milk guide channel 141. The milk guide channel 141 communicates with the negative pressure chamber 131. The milk guide channel 141 is at least configured to guide milk from a breast. The negative pressure member 13, the milk guide member 14, and the sealing cover 12 are integrally formed. One of the milk guide channel 141 and the negative pressure chamber 131 communicates with the milk storage chamber 111.

According to the above technical solution, the milk guide member 14 having the milk guide channel 141 is provided. The milk guide channel 141 communicates with a breast accommodating chamber 21 that is configured to accommodate the breast, and can guide the milk expressed from the breast. The negative pressure member 13 having the negative pressure chamber 131 is provided. The negative pressure chamber 131 communicates with the milk guide channel 141. The negative pressure chamber 131 can provide a negative pressure for the milk guide channel 141, thereby providing the negative pressure for the breast accommodating chamber 21 to express the milk. One of the negative pressure chamber 131 and the milk guide channel 141 communicates with the milk storage chamber 111, so as to deliver the milk to the milk storage chamber 111. Both the negative pressure member 13 and the milk guide member 14 are connected to the sealing cover 12, and the negative pressure member 13, the milk guide member 14, and the sealing cover 12 are integrally formed. That is, the negative pressure member 13, the milk guide member 14, and the sealing cover 12 are designed into a whole. This reduces the number of components of the breast pump assembly 10, thereby lowering the assembly difficulty of the breast pump assembly 10, and improving the assembly efficiency. In addition, the operation of assembling the negative pressure member 13 and the milk guide member 14 on the sealing cover 12 is omitted, thereby preventing the problem of poor airtightness due to assembly errors of the negative pressure member 13 and the milk guide member 14. Therefore, the breast pump assembly 10 provided by this embodiment of the present disclosure has low assembly difficulty and high assembly efficiency. It also prevents poor airtightness caused by the assembly errors of the negative pressure member 13 and the milk guide member 14, thereby eliminating potential airtightness hazards.

As an implementation, both the milk guide member 14 and the negative pressure member 13 are connected to a side of the sealing cover 12 facing the milk storage chamber 111. In this way, both the milk guide member 14 and the negative pressure member 13 are accommodated in the milk storage chamber 111, i.e., both the milk guide channel 141 and the negative pressure chamber 131 are accommodated in the milk storage chamber 111. This can effectively utilize the space of the milk storage chamber 111, reduce assembly of components outside the breast pump assembly 10, and facilitate communication of one of the milk guide channel 141 and the negative pressure chamber 131 with the milk storage chamber 111. Certainly, in specific applications, as an alternative implementation, at least one of the milk guide member 14 and the negative pressure member 13 may also be connected to a side of the sealing cover 12 away from the milk storage chamber 111.

As an implementation, referring to FIG. 4, FIG. 5, and FIG. 9, a first perforation 121 and a second perforation 122 are formed in the sealing cover 12. A central axis of the first perforation 121 is parallel to a central axis of the second perforation 122. The negative pressure member 13 is connected to the first perforation 121. The first perforation 121 is configured to communicate the negative pressure chamber 131 with the negative pressure hole 311. The milk guide member 14 is connected to the second perforation 122. The second perforation 122 communicates with the milk guide channel 141, and is configured to allow the milk to pass through and flow to the milk guide channel 141. In this way, the negative pressure member 13 and the milk guide member 14 are connected to the sealing cover 12, the milk can flow to the milk guide channel 141, and the negative pressure hole 311 communicates with the negative pressure chamber 131. In addition, the first perforation 121 communicates with the negative pressure chamber 131, and the second perforation 122 communicates with the milk guide channel 141, facilitating cleaning of the negative pressure chamber 131 via the first perforation 121, and cleaning of the milk guide channel 141 via the second perforation 122.

On the basis of the above implementation, the central axis of the first perforation 121 is coaxial with a central axis of the negative pressure chamber 131, and the central axis of the second perforation 122 is coaxial with a central axis of the milk guide channel 141. That is, the central axis of the negative pressure chamber 131 is parallel to the central axis of the milk guide channel 141, such that the negative pressure member 13 and the milk guide member 14 are arranged side by side on the side of the sealing cover 12 facing the milk storage chamber 111. In contrast to the arrangement where the milk guide member 14 and the negative pressure member 13 are crossed, this reduces the occupied space of the container body 11, thereby reducing the occupied space of the breast pump assembly 10. Specifically, both the central axis of the negative pressure chamber 131 and the central axis of the milk guide channel 141 are perpendicular to a plane where the sealing cover 12 is located. Both the negative pressure member 13 and the milk guide member 14 are perpendicular to the sealing cover 12.

As an implementation, the first perforation 121 is closely adjacent to the second perforation 122. Since the negative pressure member 13 is connected to the first perforation 121, and the milk guide member 14 is connected to the second perforation 122, by making the first perforation 121 closely adjacent to the second perforation 122, the negative pressure member 13 and the milk guide member 14 are arranged closely together, improving compactness of arrangement for the components of the breast pump assembly 10. It should be understood that in other embodiments, the first perforation 121 may also not be closely adjacent to the second perforation 122.

As an implementation, when the breast pump 100 is placed against the breast, the first perforation 121 is located under the second perforation 122. In this way, the negative pressure member 13 is disposed under the milk guide member 14, i.e., the negative pressure chamber 131 is disposed under the milk guide channel 141. In specific applications, the milk guide channel 141 is located in a middle of the breast pump 100. By disposing the negative pressure chamber 131 under the milk guide channel 141, the negative pressure chamber 131 is located on a middle lower portion of the breast pump 100. Therefore, the following pumping power device 321 is disposed on a lower portion of the breast pump 100, thereby lowering a center of gravity of the breast pump 100 and further enabling the breast pump 100 to be tightly worn on the breast. Certainly, in specific applications, when the breast pump 100 is placed against the breast, the first perforation 121 may also be located at other positions, such as a left side, a right side, and an upside, of the second perforation 122.

As an implementation, when the breast pump 100 is placed against the breast, the first perforation 121 is located directly under the second perforation 122, and the negative pressure chamber 131 communicates with the milk storage chamber 111. In this way, the negative pressure member 13 is disposed directly under the milk guide member 14, thereby reducing the occupied space for left and right sides of the container body 11, and making an external contour of the container body 11 more aesthetically-pleasing. By this time, the negative pressure member 13 serves as a three-way structure, which communicates with the negative pressure hole 311, the milk guide channel 141, and the milk storage chamber 111.

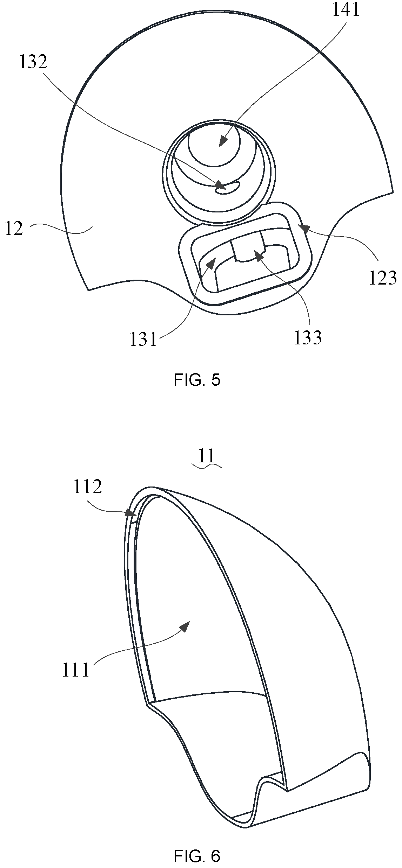

As an implementation, referring to FIGS. 5-7, the container body 11 is provided with a first mounting groove 112. The sealing cover 12 is accommodated in the first mounting groove 112, so as to enclose the milk storage chamber 111 with the container body 11. In this way, the sealing cover 12 is not convexly disposed at an opening of the container body 11, reducing a thickness of the breast pump assembly 10, and thus facilitating thinning of the breast pump 100. Certainly, in specific applications, as an alternative implementation, the container body 11 may also not be provided with the first mounting groove 112.

As an implementation, the breast pump assembly 10 further includes a sealing member (not shown in the figure). The sealing member is disposed between the sealing cover 12 and the container body 11, so as to seal the milk storage chamber 111.

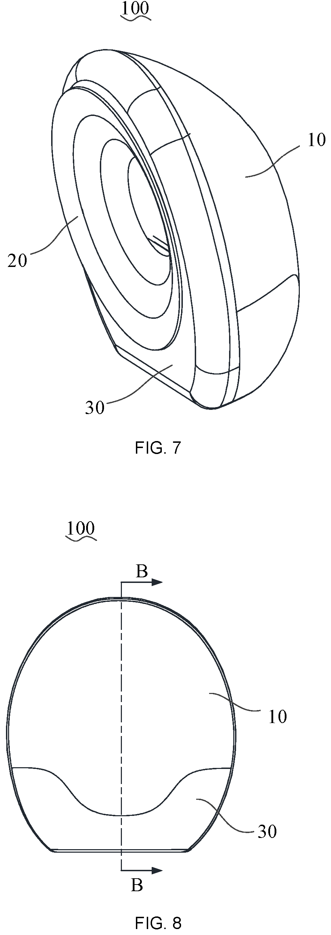

Further, referring to FIGS. 7-9, an embodiment of the present disclosure further provides a breast pump 100, including: a breast shield 20, a main unit 30, and the breast pump assembly 10. A breast accommodating chamber 21 is formed in the breast shield 20. The main unit 30 is provided with the negative pressure hole 311 configured to transfer a negative pressure. The milk guide channel 141 communicates with the breast accommodating chamber 21. The negative pressure chamber 131 communicates with the negative pressure hole 311. With the above breast pump assembly 10, the assembly efficiency of the breast pump is improved, and the situation where the airtightness is poor due to assembly errors of the negative pressure member 13 and the milk guide member 14 is reduced.

As an implementation, referring to FIG. 4 and FIGS. 9-11, the first perforation 121 is formed in the sealing cover 12. The negative pressure member 13 is connected to the first perforation 121 and located on the side of the sealing cover 12 facing the milk storage chamber 111. The breast pump 100 further includes an elastic diaphragm 40. The elastic diaphragm 40 includes a diaphragm body 41 and a turnup 42 connected to the diaphragm body 41. A circumferential direction of the main unit 30 around the negative pressure hole 311 and/or a circumferential direction of the sealing cover 12 around the first perforation 121 is provided with a second mounting groove 123. The turnup 42 is disposed in the second mounting groove 123. The diaphragm body 41 is accommodated in the negative pressure chamber 131. In this way, while the elastic diaphragm 40 is mounted, the thickness of the breast pump 100 is not increased, facilitating thinning of the breast pump 100.

For example, in this embodiment, the circumferential direction of the sealing cover 12 around the first perforation 121 is provided with the second mounting groove 123. The turnup 42 of the elastic diaphragm 40 is disposed in the second mounting groove 123. The diaphragm body 41 of the elastic diaphragm 40 is accommodated in the negative pressure chamber 131. In this way, the elastic diaphragm 40 is mounted more conveniently.

As an implementation, referring to FIG. 3, FIG. 8, and FIG. 9, the negative pressure member 13 further has a first through hole 132 and a second through hole 133. The first through hole 132 is configured to communicate the negative pressure chamber 131 with the milk guide channel 141. The second through hole 133 is configured to communicate the negative pressure chamber 131 with the milk storage chamber 111. When the breast pump 100 is placed against the breast, the negative pressure chamber 131 is located under the milk guide channel 141. The elastic diaphragm 40 has a contracted state and an expanded state. When the elastic diaphragm 40 is in the expanded state, at least a portion of the elastic diaphragm 40 corresponding to the first through hole 132 is spaced apart from a wall of the negative pressure chamber 131. In this way, when the elastic diaphragm 40 is in the expanded state, there is a certain space between the at least portion of the elastic diaphragm 40 corresponding to the first through hole 132 and the wall of the negative pressure chamber 131. This space can accommodate the milk, thereby preventing the milk from flowing back to the milk guide channel 141 through the first through hole 132 when the elastic diaphragm 40 is in the expanded state. A check valve (not shown in the figure) is disposed at the second through hole 133, so as to prevent backflow of the milk to the negative pressure chamber 131 from the milk storage chamber 111.

For example, in this embodiment, when the elastic diaphragm 40 is in the expanded state, a portion, corresponding to the first through hole 132, of a side of the elastic diaphragm 40 facing the first through hole 132 inclines to form a certain space.

As an implementation, referring to FIGS. 8-10, the main unit 30 includes a first support arm 31 and a second support arm 32. The first support arm 31 is clamped between the breast shield 20 and the breast pump assembly 10. The second support arm 32 is located on one side of the breast pump assembly 10. The negative pressure hole 311 is formed in a side of the first support arm 31 close to the second support arm 32. The second support arm 32 includes the pumping power device 321. The sealing cover 12 is disposed on a side of the container body 11 close to the first support arm 31. Specifically, when the breast pump 100 is located on the breast, the second support arm 32 is located under the breast pump assembly 10. Considering that the first perforation 121 is located under the second perforation 122, and the negative pressure member 13 is located under the milk guide member 14, a negative pressure generated by the pumping power device 321 can be quickly transferred to the negative pressure chamber 131. Meanwhile, this arrangement can reduce the thickness of the breast pump 100, such that the breast pump 100 is thinner.

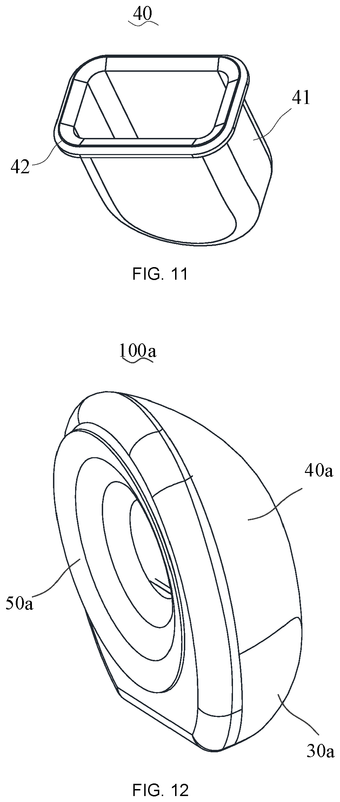

As shown in FIGS. 12-14, an embodiment of the present disclosure provides a breast pump 100a, including: a milk guide member 10a, a negative pressure member 20a, and a main unit 30a. The milk guide member 10a is made of an optically transparent material and has a milk guide channel 11a. The milk guide channel 11a is at least configured to guide milk from a breast. The negative pressure member 20a has a negative pressure chamber 21a. The negative pressure chamber 21a at least communicates with the milk guide channel 11a. The main unit 30a is provided with a negative pressure hole 31a. The negative pressure hole 31a communicates with the negative pressure chamber 21a. The main unit 30a is configured to generate a negative pressure at the negative pressure hole 31a. The negative pressure chamber 21a communicates with the negative pressure hole 31a and the milk guide channel 11a, such that a negative pressure can be generated in the milk guide channel 11a. When the breast pump 100a is placed against the breast, the negative pressure member 20a is located under the milk guide member 10a, and the main unit 30a is located at a position other than on the milk guide member 10a.

According to the above technical solution, the milk guide member 10a having the milk guide channel 11a is provided, and configured to deliver the milk from the breast, and the milk guide member 10a is made of the optically transparent material, such that the user can directly view a condition in the milk guide channel 11a. Since the negative pressure member 20a is disposed under the milk guide member 10a, and the main unit 30a is disposed at the position other than on the milk guide member 10a, when the breast pump 100a is used, the user views the breast pump 100a from the position above the breast pump 100a, and neither the negative pressure member 20a nor the main unit 30a shields the milk guide member 10a. Thus, the user can view a flowing condition of the milk in the milk guide channel 11a in real time. In case of an excessively fast or slow flow rate, an operation mode can be adjusted at any time, so as to slow down or speed up flowing of the milk. Therefore, when the breast pump 100a provided by this embodiment is used, the user can quickly and effectively get the problem of the breast pump 100a to immediately adjust the operation mode, greatly improving the use effect of the breast pump 100a.

As an implementation, at least a portion of the main unit 30a is disposed on a side of the negative pressure member 20a away from the milk guide member 10a. That is, when the breast pump 100a is placed against the breast, at least a portion of the main unit 30a is located under the negative pressure member 20a. The milk guide channel 11a is typically disposed in a middle of the breast pump 100a because it receives the milk from the breast. That is, the milk guide member 10a is disposed in the middle of the breast pump 100a. By disposing the negative pressure member 20a and at least a portion of the main unit 30a under the milk guide member 10a, a center of gravity of the breast pump 100a is lowered, enabling the breast pump 100a to be tightly worn on the breast.

For example, in this embodiment, a portion of the main unit 30a is disposed on the side of the negative pressure member 20a away from the milk guide member 10a, and a portion of the main unit 30a is disposed aside the negative pressure member 20a and the milk guide member 10a.

As an implementation, the negative pressure member 20a is made of the optically transparent material. In specific applications, when being disposed under the milk guide member 10a, the negative pressure member 20a serves as a three-way structure, and communicates with the negative pressure hole 31a, the milk guide channel 11a, and a following milk storage chamber 43a. The negative pressure chamber 21a also serves to guide the milk. Since the negative pressure member 20a is made of the optically transparent material, the user can view the interior of the negative pressure chamber 21a, thereby viewing a condition of the milk in the negative pressure chamber 21a.

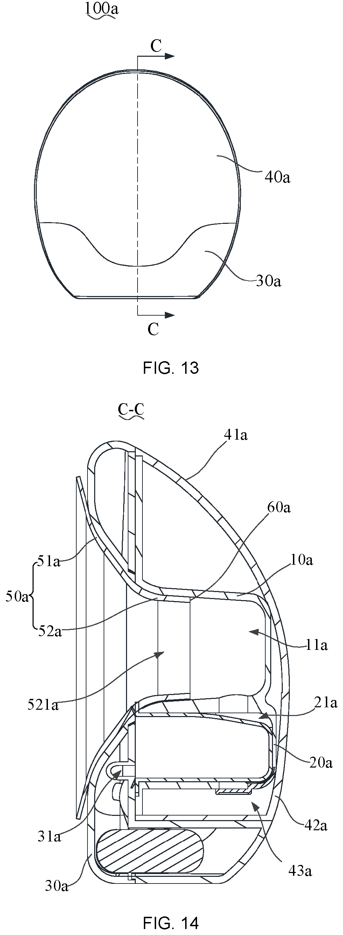

As an implementation, referring to FIG. 13 and FIG. 14, the breast pump 100a further includes a milk storage container 40a. Both the milk guide member 10a and the negative pressure member 20a are connected to an inner side of the milk storage container 40a, and the main unit 30a is disposed on an outer side of the milk storage container 40a. Certainly, in specific applications, as an alternative implementation, the breast pump 100a may also not include the milk storage container 40a. In this case, the breast pump 100a may be externally connected to a container such as a milk storage bag or a milk bottle to store the milk.

On the basis of the above implementation, when the breast pump 100a is placed against the breast, at least a portion of the milk storage container 40a located above the milk guide member 10a is made of the optically transparent material. In this way, the user can further view the interior of the milk storage container 40a, and can view the interior of the milk guide channel 11a through the milk storage container 40a from above in use.

As an implementation, the milk storage container 40a is entirely made of the optically transparent material. In this way, the user can view the interior of the milk storage container 40a from all directions. Considering that both the milk guide member 10a and the negative pressure member 20a are made of the transparent material, the user can clearly know an entire delivery condition of the milk expressed from the nipple to the milk storage container 40a.

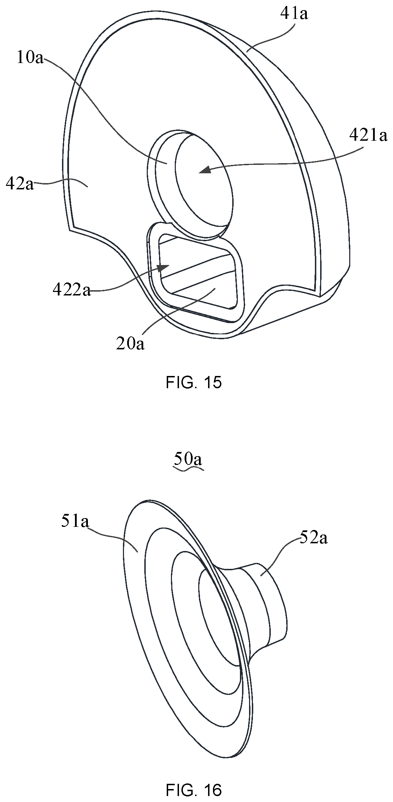

As an implementation, referring to FIGS. 13-15, the milk storage container 40a includes a first container wall 41a and a second container wall 42a. The first container wall 41a and the second container wall 42a enclose the milk storage chamber 43a. Both the negative pressure member 20a and the milk guide member 10a are connected to a side of the second container wall 42a facing the milk storage chamber 43a. When the breast pump 100a is placed against the breast, the first container wall 41a is located above the milk guide member 10a. Specifically, each of the first container wall 41a and the second container wall 42a has a hollow cavity. After the first container wall 41a and the second container wall 42a are connected, their cavities form the milk storage chamber 43a. A first perforation 421a and a second perforation 422a are formed in the second container wall 42a. Both the first perforation 421a and the second perforation 422a communicate with the milk storage chamber 43a. The milk guide member 10a is connected to the first perforation 421a. The first perforation 421a is configured to communicate the milk guide channel 11a with an exterior of the milk storage container 40a, such that the milk can be guided to the milk guide channel 11a. The negative pressure member 20a is connected to the second perforation 422a. The second perforation 422a is configured to communicate the negative pressure chamber 21a with the negative pressure hole 31a.

On the basis of the above implementation, transparency of the first container wall 41a is greater than or equal to transparency of the second container wall 42a. In this way, the user can clearly view the interior of the milk storage container 40a when using the breast pump 100a.

As an implementation, referring to FIG. 12 and FIGS. 14-16, the breast pump 100a further includes a breast shield 50a. The breast shield 50a includes a breast attachment portion 51a and a channel portion 52a connected to the breast attachment portion 51a. The breast attachment portion 51a is configured to be adsorbed to the breast. The channel portion 52a is provided with a nipple channel 521a. At least a portion of the channel portion 52a extends into the milk guide member 10a, such that the nipple channel 521a communicates with the milk guide channel 11a. Specifically, the channel portion 52a extends through the first perforation 421a into the milk guide channel 11a of the milk guide member 10a. A nipple is accommodated in the nipple channel 521a. The milk expressed from the nipple flows from the nipple channel 521a to the milk guide channel 11a.

As an implementation, the channel portion 52a is made of the optically transparent material. In this way, the user can view the interior of the nipple channel 521a. When the breast pump 100a is worn on the breast, the user can check whether the nipple is aligned with the nipple channel 521a and whether the nipple is accurately accommodated in the nipple channel 521a, thereby improving the wearing efficiency of the breast pump 100a on the breast.

As an implementation, the breast attachment portion 51a and the channel portion 52a are integrally formed. This reduces the assembly steps of the breast shield 50a, and effectively ensures the sealing performance of connection between the breast attachment portion 51a and the channel portion 52a. Certainly, in specific applications, as an alternative implementation, the breast attachment portion 51a and the channel portion 52a may also be manufactured individually for assembly.

On the basis of the above implementation, the breast attachment portion 51a is made of the optically transparent material.

As an implementation, the channel portion 52a is in an interference fit with the milk guide member 10a. In this way, the channel portion 52a is connected to the milk guide member 10a conveniently and quickly.

As an implementation, the breast shield 50a is detachably connected to the milk guide member 10a. Upon completion of milk expression, the breast shield 50a can be detached from the milk guide member 10a, facilitating the cleaning of the breast shield 50a and the milk guide member 10a.

As an implementation, an outer surface of the channel portion 52a is smooth, which is structurally simple, and easy to manufacture.

As an implementation, referring to FIG. 14, an end of the channel portion 52a away from the breast attachment portion 51a and an inner surface of the milk guide member 10a define a step 60a, so as to prevent backflow of the milk. Specifically, the milk comes into contact with the step 60a when flowing back, and the step 60a obstructs the milk, preventing further flowing of the milk to the breast attachment portion 51.

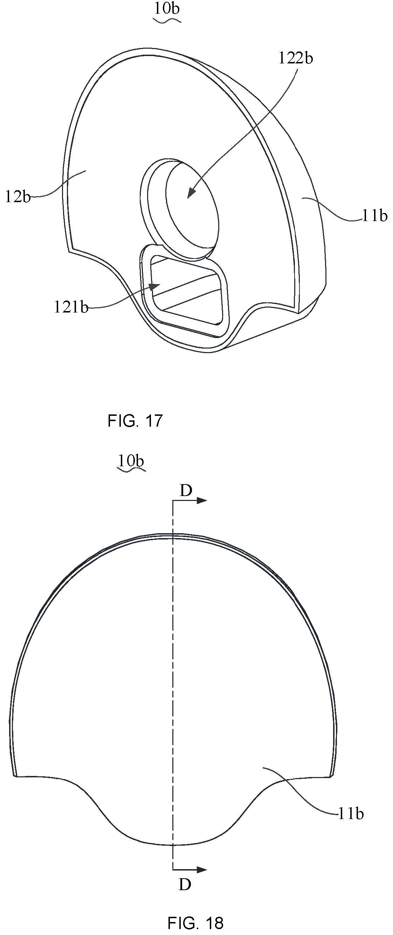

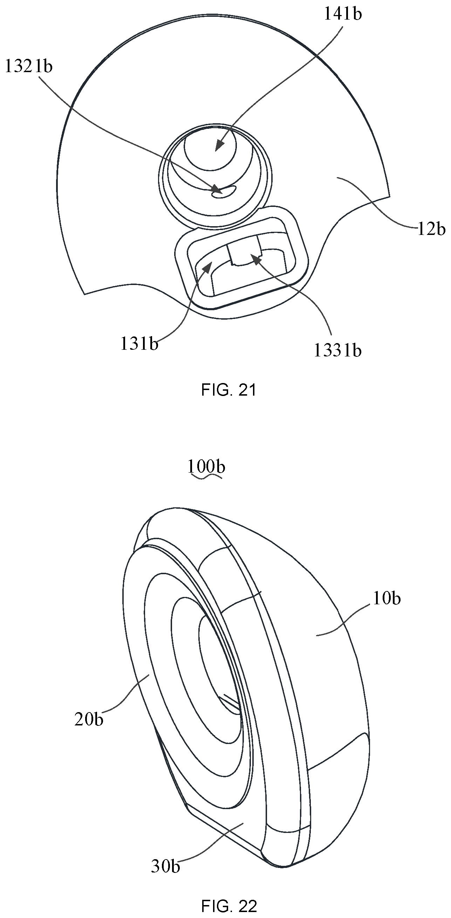

As shown in FIG. 17 and FIG. 22, an embodiment of the present disclosure provides a breast pump assembly 10b for a breast pump 100b. The breast pump 100b can be worn on a breast, which frees the user's hands and allows the user to perform other tasks while using the breast pump 100b for milk expression. The breast pump assembly 10b is at least configured to express the milk.

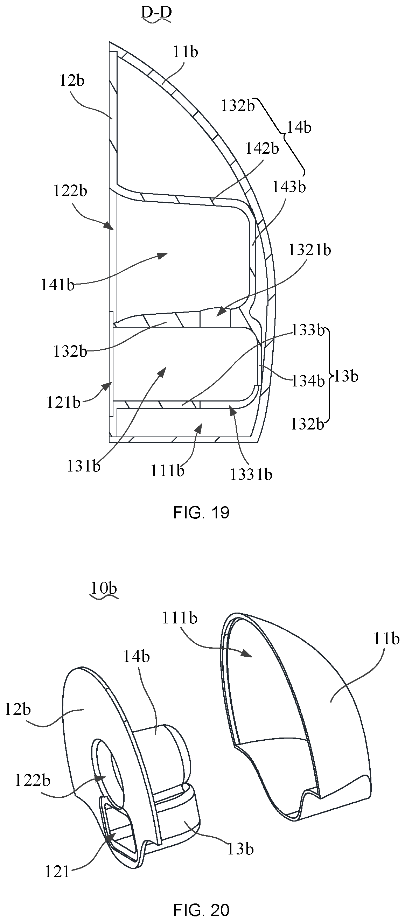

Referring to FIG. 17 and FIG. 20, the breast pump assembly 10b includes a container body 11b and a sealing cover 12b. The container body 11b and the sealing cover 12b enclose a milk storage chamber 111b. In this way, the milk can be temporarily stored in the milk storage chamber 111b well.

As an implementation, referring to FIGS. 18-20, FIG. 23, and FIG. 24, the breast pump assembly 10b further includes a negative pressure member 13b and a milk guide member 14b. The negative pressure member 13b has a negative pressure chamber 131b. The negative pressure chamber 131b is configured to communicate with a negative pressure hole 311b of the breast pump 100b. The negative pressure hole 311b is configured to provide a negative pressure for the negative pressure chamber 131b. The milk guide member 14b has a milk guide channel 141b. The milk guide channel 141b communicates with the negative pressure chamber 131b. The milk guide channel 141b is at least configured to guide milk from a breast. The milk guide member 14b and the negative pressure member 13b are arranged side by side in the breast pump 100b.

According to the above technical solution, the milk guide member 14b having the milk guide channel 141b is provided. The milk guide channel 141b communicates with a breast accommodating chamber 21b that is configured to accommodate the breast, and can be configured to guide the milk expressed from the breast. The negative pressure member 13b having the negative pressure chamber 131b is provided. The negative pressure chamber 131b communicates with the milk guide channel 141b. The negative pressure chamber 131b can be configured to provide the negative pressure for the milk guide channel 141b, thereby providing a negative pressure for the breast accommodating chamber 21b, and thus expressing the milk. The milk guide member 14b and the negative pressure member 13b are arranged side by side in the breast pump 100b, i.e., the milk guide member 14b and the negative pressure member 13b have a same extension direction, so the breast pump 100b only needs to provide an enough space in a direction same as the extension direction of the milk guide member 14b and the negative pressure member 13b to accommodate the milk guide member 14b and the negative pressure member 13b. In contrast to the arrangement where the milk guide member 14b and the negative pressure member 13b are crossed, and the breast pump 100b provides enough accommodating spaces in two different extension directions for the milk guide member 14b and the negative pressure member 13b, the occupied space of the breast pump assembly 10b is effectively reduced. Therefore, the breast pump assembly 10b provided by this embodiment has a small occupied space, thereby reducing the overall size of the breast pump 100b.

As an implementation, the milk guide member 14 and the negative pressure member 13 are connected side by side to a same surface of the sealing cover 12 and extend toward one side of the milk storage chamber 111. This can effectively utilize the space of the milk storage chamber 111b, reducing assembly of external components for the breast pump assembly 10b.

As an implementation, when the breast pump 100b is placed against the breast, the negative pressure member 13b is located under the milk guide member 14b. By this time, the negative pressure chamber 131b is located under the milk guide channel 141b. In specific applications, the milk guide channel 141b is located in a middle of the breast pump 100b. In this way, the negative pressure chamber 131b is located on a middle lower portion of the breast pump 100b. Therefore, the following pumping power device 321b is disposed on a lower portion of the breast pump 100b, thereby lowering a center of gravity of the breast pump 100b and further enabling the breast pump 100b to be tightly worn on the breast. Certainly, in specific applications, when the breast pump 100b is placed against the breast, the negative pressure member 13b may also be located at other positions, such as a left side, a right side, and an upside, of the milk guide member 14b.

On the basis of the above implementation, when the breast pump 100b is placed against the breast, the negative pressure member 13b is located directly under the milk guide member 14b, and the negative pressure chamber 131b communicates with the milk storage chamber 111b. This reduces the occupied space for left and right sides of the container body 11b, and makes an external contour of the container body 11b more aesthetically-pleasing. By this time, the negative pressure member 13b serves as a three-way structure, which communicates with the negative pressure hole 311b, the milk guide channel 141b, and the milk storage chamber 111b.

As an implementation, referring to FIG. 20, the milk guide member 14b and the negative pressure member 13b share a first side wall 132b. Two opposite sides of the first side wall 132b face the negative pressure chamber 131b and the milk guide channel 141b, respectively. A first through hole 1321b configured to communicate the negative pressure chamber 131b with the milk guide channel 141b is formed in the first side wall 132b. This simplifies the communication structure between the milk guide member 14b and the negative pressure member 13b, improving the production efficiency. Certainly, in specific applications, as an alternative implementation, the milk guide member 14b and the negative pressure member 13b may also be spaced apart, and the negative pressure chamber 131b and the milk guide channel 141b may also communicate via a communication tube.

As an implementation, when the breast pump 100b is placed against the breast, the first through hole 1321b is located at a lowest position of the milk guide member 14b. The milk in the milk guide channel 141b flows out from the first through hole 1321b under the action of gravity. By forming the first through hole 1321b at the lowest position of the milk guide member 14b, the milk in the milk guide channel 141b flows out from the first through hole 1321b as much as possible, thereby reducing milk accumulation in the milk guide channel 141b.

As an implementation, referring to FIG. 19, FIG. 21, FIG. 22, and FIG. 24, the negative pressure member 13b further includes a second side wall 133b and a first bottom wall 134b. The first side wall 132b, the second side wall 133b, and the first bottom wall 134b enclose the negative pressure chamber 131b. A second through hole 1331b configured to communicate the negative pressure chamber 131b with the milk storage chamber 111b is formed in the second side wall 133b. When the breast pump 100b is placed against the breast, the second through hole 1331b is located under the first through hole 1321b. In this way, the milk flows from the negative pressure chamber 131b to the milk storage chamber 111b. The first bottom wall 134b is opposite to an opening of the negative pressure chamber 131b. The opening of the negative pressure chamber 131b communicates with the negative pressure hole 311b.

As an implementation, when the breast pump 100b is placed against the breast, the second through hole 1331b is located at a lowest position of the negative pressure member 13b. The milk in the negative pressure chamber 131b flows out from the second through hole 1331b under the action of gravity. By forming the second through hole 1331b at the lowest position of the negative pressure member 13b, the milk in the negative pressure chamber 131b flows out from the second through hole 1331b as much as possible, thereby reducing milk accumulation in the negative pressure chamber 131b.

As an implementation, when the breast pump 100b is placed against the breast, the second through hole 1331b directly faces the first through hole 1321b along a vertical direction. In this way, the milk entering the negative pressure chamber 131b through the first through hole 1321b can directly enter the milk storage chamber 111b through the second through hole 1331b, reducing a flow path of the milk in the negative pressure chamber 131b, and shortening retention time of the milk in the negative pressure chamber 131b.

In another implementation, when the breast pump 100b is placed against the breast, the second through hole 1331b is formed in an end of the second side wall 133b close to the first bottom wall 134b. In specific applications, a diaphragm 40b is disposed in the negative pressure chamber 131b. The diaphragm 40b has a contracted state and an expanded state. When the diaphragm 40b located in the negative pressure chamber 131b is in the expanded state, the diaphragm 40b expands from the opening of the negative pressure chamber 131b to the first bottom wall 134b. By forming the second through hole 1331b in the end of the second side wall 133b close to the first bottom wall 134b, the diaphragm 40b can push the milk to the second through hole 1331b during expansion, such that the milk flows out from the second through hole 1331b, reducing accumulation of the milk in the negative pressure chamber 131b.

As an implementation, the first bottom wall 134b is in smooth transition with the first side wall 132b and the second side wall 133b. This prevents dead angles at junctions of the first bottom wall 134b with the first side wall 132b and the second side wall 133b, facilitating the cleaning of the negative pressure chamber 131b.

As an implementation, referring to FIG. 19, the milk guide member 14b further includes a third side wall 142b and a second bottom wall 143b. The first side wall 132b, the third side wall 142b, and the second bottom wall 143b enclose the milk guide channel 141b. The second bottom wall 143b is opposite to an entrance of the milk guide channel 141b.

On the basis of the above implementation, the second bottom wall 143b is in smooth transition with the first side wall 132b and the third side wall 142b. This prevents dead angles at junctions of the second bottom wall 143b with the first side wall 132b and the third side wall 142b, facilitating the cleaning of the milk guide channel 141b.

As an implementation, a length of the milk guide member 14b is the same as a length of the negative pressure member 13b. In specific applications, the milk guide member 14b and the negative pressure member 13b extend along a thickness direction of the breast pump 100b, with the same length. This reduces the thickness of the breast pump 100b, and avoids the situation where either member is excessively long to cause the overlarge thickness of the breast pump 100b.

As an implementation, an opening of the negative pressure chamber 131b for communicating with the negative pressure hole 311b and an opening of the milk guide channel 141b for receiving the milk are arranged side by side and have a same orientation. In this way, for the breast pump 100b, the main unit 30b provided with the negative pressure hole 311b and the breast shield 20b configured to deliver the milk to the milk guide channel 141b are located on a same side of the breast pump assembly 10b, such that the main unit 30b, the breast shield 20b, and the breast pump assembly 10b are arranged more compactly, thereby reducing the overall size.

As an implementation, referring to FIG. 20, FIG. 21, and FIG. 24, a first perforation 121b and a second perforation 122b are formed in the sealing cover 12b. A central axis of the first perforation 121b and a central axis of the second perforation 122b are parallel or approximately parallel, with an included angle not greater than 20°. The negative pressure member 13b is connected to the first perforation 121b. The first perforation 121b is configured to communicate the negative pressure chamber 131b with the negative pressure hole 311b. The milk guide member 14b is connected to the second perforation 122b. The second perforation 122b communicates with the milk guide channel 141b, and is configured to allow the milk to pass through and flow to the milk guide channel 141b. In this way, the negative pressure member 13b and the milk guide member 14b are connected to the sealing cover 12b, the milk can flow to the milk guide channel 141b, and the negative pressure hole 311b communicates with the negative pressure chamber 131b. Specifically, both the central axis of the first perforation 121b and the central axis of the second perforation 122b are perpendicular or approximately perpendicular to a plane where the sealing cover 12b is located, and form an included angle of 80-100° with the plane where the sealing cover 12b is located. Both the negative pressure member 13b and the milk guide member 14b are perpendicular or approximately perpendicular to the sealing cover 12b. The negative pressure member 13b and the milk guide member 14b each form an included angle of 80-100° with the sealing cover 12b.

Further, referring to FIGS. 22-24, an embodiment further provides a breast pump 100b, including: a breast shield 20b, a main unit 30b, and the breast pump assembly 10b. A breast accommodating chamber 21b is formed in the breast shield 20b. The main unit 30b is provided with a negative pressure hole 311b configured to transfer a negative pressure. The milk guide channel 141b communicates with the breast accommodating chamber 21b. The negative pressure chamber 131b communicates with the negative pressure hole 311b. With the above breast pump assembly 10b, the size of the breast pump 100b is reduced, which is convenient for the user to carry and use.

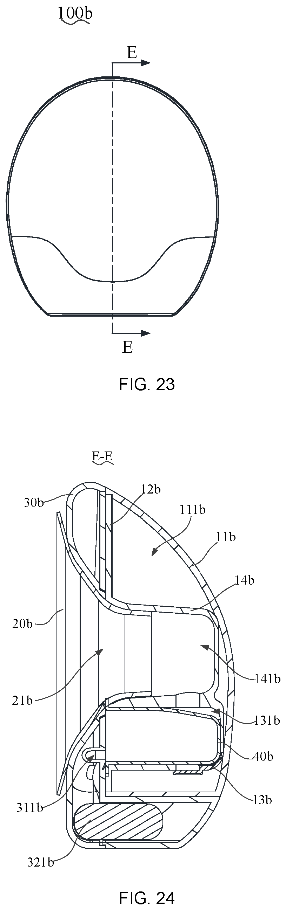

As an implementation, referring to FIGS. 23-25, the main unit 30b includes a first support arm 31b and a second support arm 32b. The first support arm 31b is clamped between the breast shield 20b and the breast pump assembly 10b. The second support arm 32b is located on one side of the breast pump assembly 10b. The negative pressure hole 311b is formed in a side of the first support arm 31b close to the second support arm 32b. The second support arm 32b includes a pumping power device 321b. Specifically, when the breast pump 100b is located on the breast, the second support arm 32b is located under the breast pump assembly 10b. Considering that the negative pressure member 13b is located under the milk guide member 14b, a negative pressure generated by the pumping power device 321b can be quickly transferred to the negative pressure chamber 131b. Meanwhile, this arrangement can reduce the thickness of the breast pump 100b, such that the breast pump 100b is thinner.

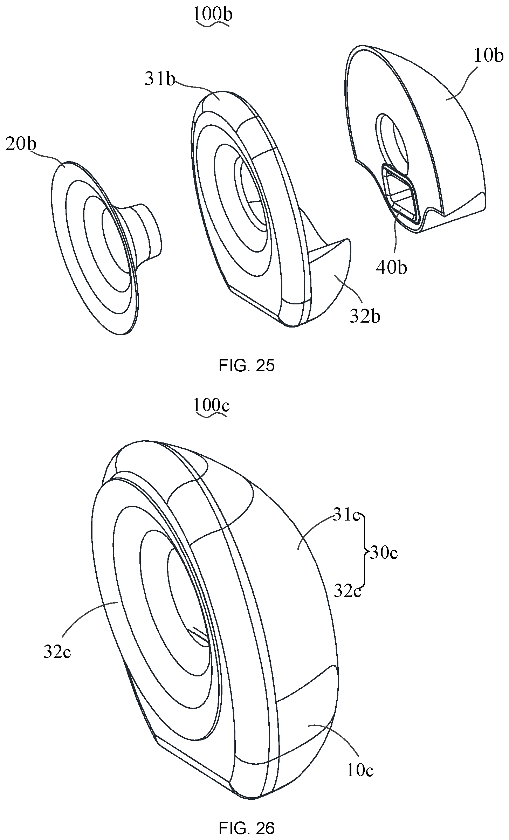

As an implementation, referring to FIG. 24 and FIG. 25, the breast pump 100b further includes a diaphragm 40b. The diaphragm 40b is connected to the sealing cover 12b and accommodated in the negative pressure chamber 131b. The diaphragm 40b is at least configured to prevent the milk from flowing to the negative pressure hole 311b.

The main unit of the breast pump is mainly configured to provide the negative pressure. In the related art, the structure of the main unit is designed unreasonably with the large occupied space, such that the breast pump has an overlarge overall size and is inconvenient to carry and use, affecting the user experience.

In view of this, an embodiment of the present disclosure provides a breast pump. By reasonably arranging a structure of the main unit, this embodiment effectively reduces the occupied space.

As shown in FIGS. 26-30, the breast pump 100c provided by this embodiment of the present disclosure includes: a main unit housing 10c, a pumping power device 20c, and a milk pumping assembly 30c. The main unit housing 10c includes a first shell 12c and a second shell 13c extending relative to the first shell 12c in a bending manner, so as to form a step 14c on the main unit housing 10c. An accommodating chamber 11c is formed in the main unit housing 10c. The pumping power device 20c is disposed in the accommodating chamber 11c, and configured to generate a negative pressure. The milk pumping assembly 30c is at least partially located on the step 14c.

The pumping power device 20c is disposed in the accommodating chamber 11c of the main unit housing 10c, so the main unit housing 10c serves to support and protect the pumping power device 20c.

According to the above technical solution, the second shell 13c extends relative to the first shell 12c in the bending manner, the step 14c is formed on the main unit housing 10c, and the milk pumping assembly 30c is at least partially located on the step 14c, such that the main unit housing 10c and the milk pumping assembly 30c are closely assembled together. This arrangement is reasonable, and greatly reduces the occupied space of the main unit housing 10c and the overall size of the breast pump 100c, thereby making the breast pump 100c convenient to carry and use, and enhancing the user experience.

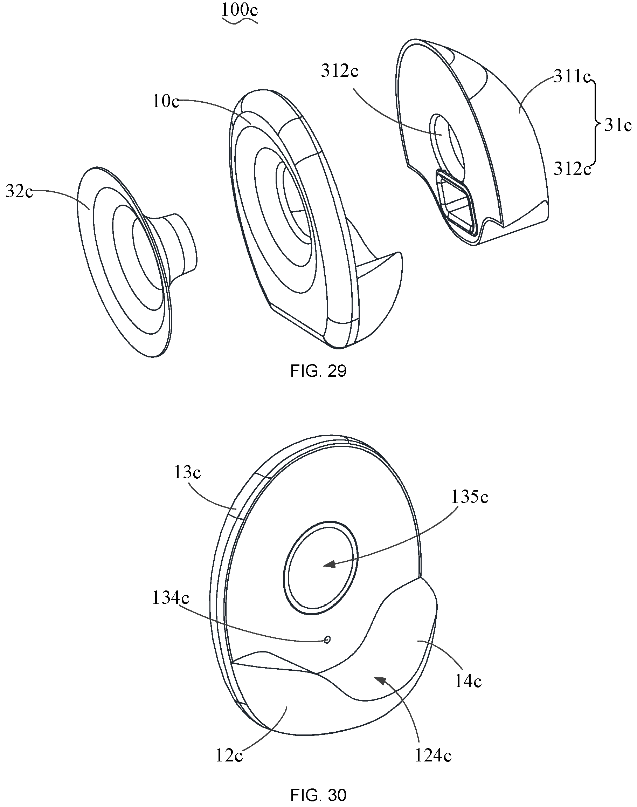

As an implementation, referring to FIGS. 29-31, the main unit housing 10c is L-shaped, and an L-shaped step 14c is formed on the main unit housing 10c. The L-shaped main unit housing 10c features a neat and regular structure with a small occupied space, and the L-shaped step 14c facilitates the assembly of a breast shield 32c and a milk collection unit 31c, such that all components of the breast pump 100c can be tightly assembled, thereby achieving a reasonable arrangement, and effectively reducing the overall size of the breast pump 100c.

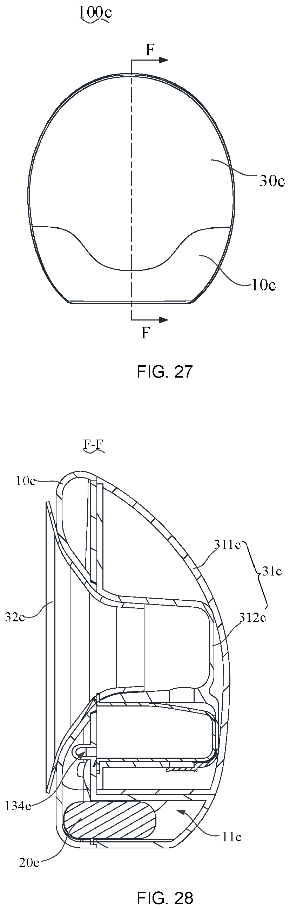

As an implementation, referring to FIG. 27, FIG. 28 and FIG. 31, the milk pumping assembly 30c includes the milk collection unit 31c and the breast shield 32c. The milk collection unit 31c is disposed on one side of the first shell 12c. The second shell 13c is disposed between the breast shield 32c and the milk collection unit 31c. At least one of the first shell 12c and the second shell 13c has a cavity, so as to form the accommodating chamber 11c. By clamping the second shell 13c between the breast shield 32c and the milk collection unit 31c, the breast shield 32c is connected to the milk collection unit 31c. The first shell 12c is disposed on one side, such as an upside, an underside, a left side or a right side, of the milk collection unit 31c. Terms such as the upside, the underside, the left side, and the right side are positions when the breast pump 100c is in an operating state.

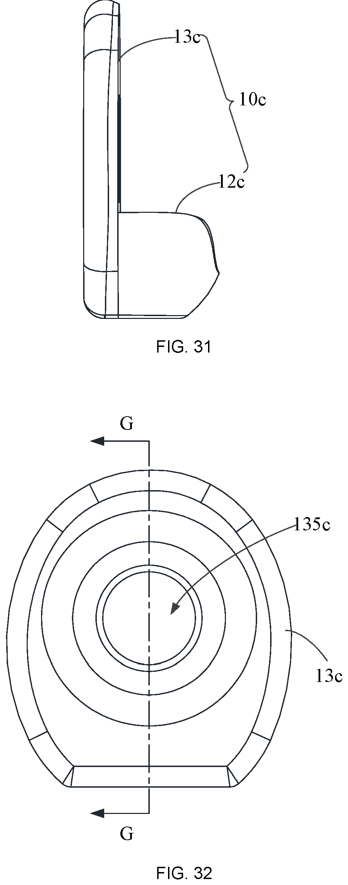

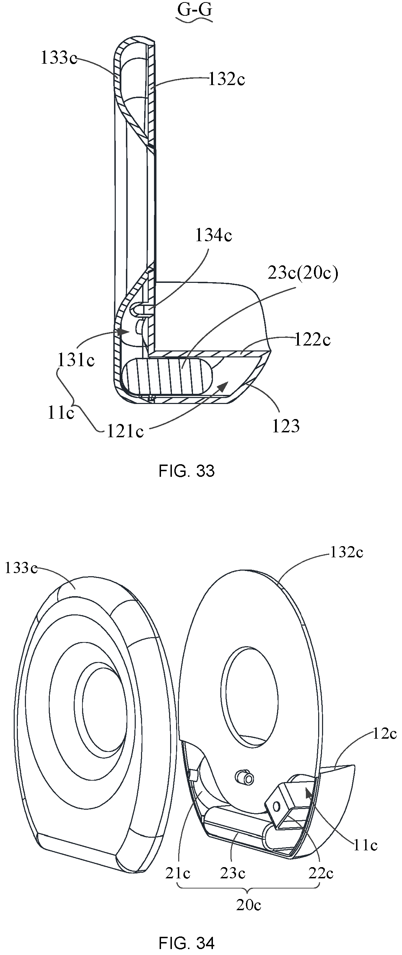

On the basis of the above implementation, the milk collection unit 31c is supported on the first shell 12c. That is, the first shell 12c is disposed on the underside of the milk collection unit 31c, i.e., when the breast pump 100c is used, the first shell 12c is located under the milk collection unit 31c. In this way, the first shell 12c can support the milk collection unit 31c, improving stability of the milk collection unit 31c. As an implementation, referring to FIGS. 31-34, the first shell 12c has a first cavity 121c. The second shell 13c has a second cavity 131c. The accommodating chamber 11c includes the first cavity 121c and the second cavity 131c. In this way, both the first cavity 121c and the second cavity 131c can accommodate the component, achieving reasonable arrangement of the pumping power device 20c, and minimizing the occupied space of the main unit housing 10c. Certainly, in specific applications, it is also appreciated that the second cavity 131c is not provided, but the second cavity 121c forms the accommodating chamber 11c, or the first cavity 121c is not provided, but the second cavity 131c forms the accommodating chamber 11c.

On the basis of the above implementation, at least a portion of the pumping power device 20c is accommodated in the first cavity 121c. This reduces the size of the second cavity 131c, the thickness of the second shell 13c, and the overall thickness of the breast pump 100c, thereby making the breast pump 100c thinner.

As an implementation, referring to FIG. 26, FIG. 31 and FIG. 36, the pumping power device 20c includes an air pump 21c, a solenoid valve 22c, a battery 23c, and a circuit board (not shown in the figure). The air pump 21c, the solenoid valve 22c, and the battery 23c are accommodated in the first cavity 121c. The circuit board is accommodated in the second cavity 131c. In this way, the weight of the pumping power device 20c is largely concentrated in the first cavity 121c. When the breast pump 100c is used, and the first shell 12c is located under the milk collection unit 31c, an overall center of gravity of the breast pump 100c is lowered, thereby achieving better sealing performance when the breast pump 100c is worn. Moreover, most components of the pumping power device 20c are disposed in the first cavity 121c, which can reduce the size of the second cavity 131c and the thickness of the second shell 13c, thereby making the overall breast pump 100c thinner.

In specific applications, any one or two of the air pump 21c, the solenoid valve 22c, and the battery 23c are accommodated in the second cavity 131c, or the circuit board is accommodated in the first cavity 121c.

The battery 23c is electrically connected to the circuit board. The circuit board is electrically connected to the air pump 21c and the solenoid valve 22c. When the air pump 21c is in operation, the solenoid valve 22c is idle, and the air pump 21c draws air out to generate a negative pressure. When the solenoid valve 22c is in operation, the air pump 21c is idle, and the solenoid valve 22c is opened, such that the air can be charged to a breast pump assembly 312c of the milk collection unit 31c.

On the basis of the above implementation, the air pump 21c, the battery 23c, and the solenoid valve 22c are arranged side by side. The battery 23c is located between the air pump 21c and the solenoid valve 22c. Both the air pump 21c and the solenoid valve 22c generate heat in operation. The air pump 21c and the solenoid valve 22c are spaced apart by the battery 23c, so as to prevent an excessive temperature in a local region. Certainly, in specific applications, as an alternative implementation, it is appreciated that the air pump 21c is disposed between the battery 23c and the solenoid valve 22c, or the solenoid valve 22c is disposed between the battery 23c and the air pump 21c.

As an implementation, referring to FIG. 29, FIG. 31, and FIG. 33, the first shell 12c includes a first top shell 122c and a first bottom shell 123c. The first top shell 122c and the first bottom shell 123c enclose the first cavity 121c. The second shell 13c includes a second top shell 132c and a second bottom shell 133c. The second top shell 132c and the second bottom shell 133c enclose the second cavity 131c. The first top shell 122c and the second top shell 132c are perpendicular or approximately perpendicular and are respectively located on two sides of the milk collection unit 31c.

As an implementation, referring to FIG. 29, FIG. 30, FIG. 33, and FIG. 34, a negative pressure hole 134c is formed in a side of the second shell 13c provided with the first shell 12c. The negative pressure hole 134c communicates with the second cavity 131c. That is, a side of the second shell 13c facing the milk collection unit 31c is provided with the negative pressure hole 134c, i.e., the second shell 132c is provided with the negative pressure hole 134c. In specific applications, the air pump 21c and the solenoid valve 22c communicate with one end of an air guide tube. Another end of the air guide tube communicates with the negative pressure hole 134c or penetrates into the negative pressure hole 134c. The negative pressure hole 134c can be configured to provide a negative pressure for the breast pump assembly 312c of the milk collection unit 31c or transfer air to it. By forming the negative pressure hole 134c in the second top shell 132c, the negative pressure is provided for the breast pump assembly 312c of the milk collection unit 31c or the air is transferred to it.

On the basis of the above implementation, the negative pressure hole 134c is formed in an end of the second shell 13c close to the first shell 12c. Both the air pump 21c and the solenoid valve 22c are disposed in the first cavity 121c of the first shell 12c. One end of the air guide tube communicates with the air pump 21c and the solenoid valve 22c. Another end of the air guide tube communicates with the negative pressure hole 134c or penetrates into the negative pressure hole 134c. The negative pressure hole 134c is formed in the end of the second shell 13c close to the first shell 12c. This can shorten the air guide tube, and enhance the neatness in the accommodating chamber 11c.

As an implementation, the main unit of the breast pump 100c further includes a key unit. The key unit is disposed on a circumferential side, other than an end close to the first shell 12c, of the second shell 13c. Disposing the key unit on the circumferential side of the second shell 13c facilitates the key operation of the user. The key unit is electrically connected to the circuit board. By operating different keys on the key unit, such parameters as a pumping frequency, a single pumping duration, and a pumping force in pumping operation can be adjusted or the on-off state of the breast pump can be operated, thereby meeting different milk pumping requirements of different users.

On the basis of the above implementation, the key unit is disposed on an end of the second shell 13c away from the first shell 12c. When the breast pump 100c is used, the first shell 12c is located on a bottom of the breast pump 100c, and the end of the second shell 13c away from the first shell 12c is located on a top of the breast pump 100c. By disposing the key unit on the end of the second shell 13c away from the first shell 12c, i.e., disposing the key unit on the end of the breast pump 100c, the key operation of the user is more convenient.

As an implementation, referring to FIG. 29 and FIG. 30, the second shell 13c is further provided with a through hole 135c. The through hole 135c extends from a side of the second shell 13c away from the first shell 12c to a side of the second shell 13c close to the first shell 12c. The through hole 135c is configured to allow the breast shield 32c to penetrate through. With the through hole 135c, the breast shield 32c and the milk collection unit 31c respectively located on two sides of the second shell 13c are connected. In specific applications, the breast shield 32c includes a breast attachment portion and a nipple channel. The through hole 135c corresponds to the nipple channel, so as to allow the nipple channel to penetrate through.

As an implementation, referring to FIG. 29, FIG. 30, and FIG. 33, a side of the first shell 12c provided with the second shell 13c is provided with a limiting recess 124c. That is, a side of the first shell 12c facing the milk collection unit 31c is provided with the limiting recess 124c, i.e., the first top shell 122c forms the limiting recess 124c. The limiting recess 124c is configured to limit the milk collection unit 31c, so as to enhance mounting stability of the milk collection unit 31c.

As an implementation, referring to FIG. 28 and FIG. 29, the milk collection unit 31c includes a milk storage container 311c and the breast pump assembly 312c extending into the milk storage container 311c. The pumping power device 20c is configured to provide the negative pressure for the breast pump assembly 312c. The breast pump assembly 312c extends into the milk storage container 311c, such that the milk collection unit 31c is arranged compactly, reducing the overall size of the breast pump 100c.

In the related art, heat sinks are used by the heat pump to dissipate heat of the main unit. The heat sinks are mostly sheet-like structures made of aluminum alloy, brass or bronze. In order to improve the heat dissipation effect, a plurality of heat sinks are to be assembled, resulting in relatively large size and weight of the breast pump to greatly compromise the user experience.

In view of this, an embodiment of the present disclosure provides a breast pump. The breast pump makes use of milk to dissipate heat of the main unit, and eliminates the heat sinks.

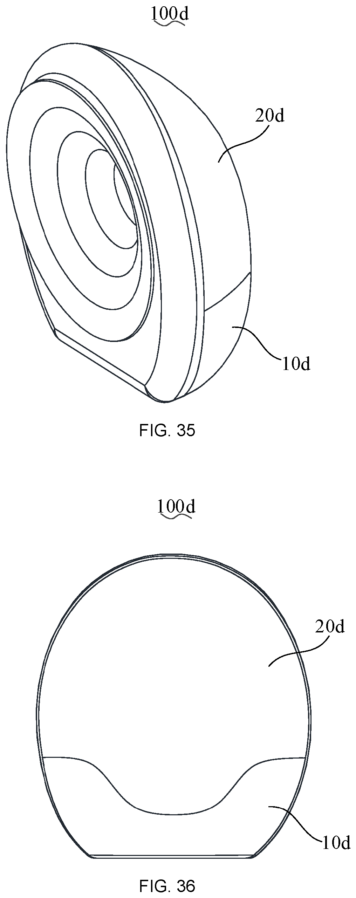

As shown in FIG. 35 and FIG. 36, the breast pump 100d provided by this embodiment of the present disclosure includes a main unit 10d and a milk storage container 20d. The main unit 10d is configured to provide a negative pressure. The milk storage container 20d is configured to store expressed milk.

In use, the main unit 10d generates the negative pressure, and a breast pump assembly of the breast pump 100d expresses milk from a breast of a user through the negative pressure, and guide the milk to the milk storage container 20d for temporary storage.

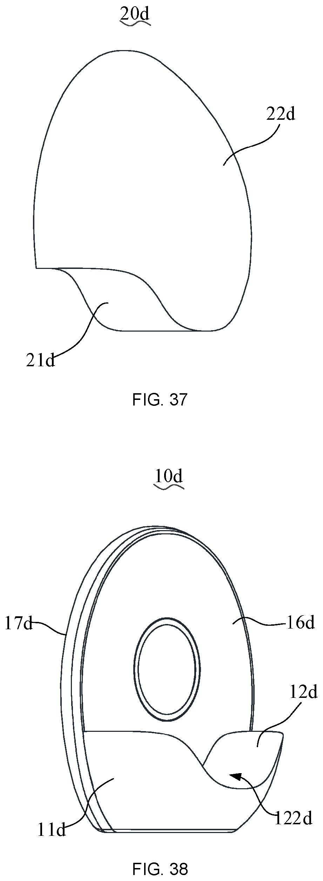

As an implementation, referring to FIG. 36 and FIG. 37, the milk storage container 20d includes a support wall 21d and an enclosure wall 22d. The support wall 21d and the enclosure wall 22d enclose a milk storage chamber (not shown in the figure). The support wall 21d is configured to support milk flowing to the milk storage chamber. In specific applications, the expressed milk flows to the milk storage chamber of the milk storage container 20d for temporary storage. When the breast pump 100d is used, the support wall 21d is located under the enclosure wall 22d. The milk entering the milk storage chamber flows to the support wall 21d under the action of gravity, and is supported on the support wall 21d.

As an implementation, referring to FIG. 36 and FIG. 37, at least a portion of the main unit 10d is attached to a side of the support wall 21d away from the milk storage chamber. That is, when the breast pump 100d is used, at least a portion of the main unit 10d is located under the support wall 21d, and attached to the support wall 21d. In this way, when the milk flows to the milk storage chamber, travels downward to the support wall 21d, and is supported on the support wall 21d, since a temperature of the milk is less than a temperature of the main unit 10d (the milk has the temperature of about 50-60°C, and the main unit 10d has the temperature of far greater than 60°C), the milk can absorb heat of the main unit 10d, and serve to dissipate the heat of the main unit 10d. In contrast to the heat dissipation method using the heat sinks, this omits the heat sinks, greatly reducing the overall size and weight of the device and thus enhancing the user experience. In addition, the main unit 10d is disposed under the milk storage container 20d, serving to support the milk storage container 20d.

As an implementation, referring to FIGS. 37-40, the main unit 10d includes a first bottom shell 11d, a first top shell 12d, and an air pump 13d. The first bottom shell 11d and the first top shell 12d enclose a first accommodating chamber 14d. The air pump 13d is accommodated in the first accommodating chamber 14d. The first top shell 12d is attached to the side of the support wall 21d away from the milk storage chamber. The air pump 13d generates a large amount of heat during operation. In this way, the milk can absorb the heat of the air pump 13d, and serve to dissipate the heat of the air pump 13d, effectively prolonging the service life of the air pump 13d.

The first top shell 12d is made of a material with a certain strength and an excellent thermal conductivity, such as thermal conductive plastic. In this way, the first top shell 12d can not only support the milk storage container 20d well, but also transfer heat efficiently.

As an implementation, a projected contour of the first top shell 12d along a direction toward the support wall 21d falls within a contour of the support wall 21d. In this way, the entire first top shell 12d is attached to the support wall 21d, maximizing a heat transfer area of the first top shell 12d to improve heat transfer efficiency. In this embodiment, the projected contour of the first top shell 12d along the direction toward the support wall 21d coincides with the contour of the support wall 21d.

As an implementation, a thickness of the first top shell 12d is less than a thickness of the first bottom shell 11d. The first top shell 12d with the smaller thickness increases the heat transfer efficiency of the first top shell 12d, while the first bottom shell 11d with the larger thickness greatly enhances the support performance of the first bottom shell 11d. Certainly, in specific applications, as an alternative, the thickness of the first top shell 12d may also be the same as the thickness of the first bottom shell 11d.

As an implementation, referring to FIG. 35, FIG. 38, and FIG. 41, the first top shell 12d includes at least two panels 121d. Any two adjacent ones of the panels 121d are disposed at an included angle. The included angle is less than 180°. In this way, the surface area of the first top shell 12d per unit lateral area of the breast pump 100d is increased, i.e., the heat transfer area per unit lateral area of the breast pump 100d is increased, thereby improving the heat transfer efficiency.

On the basis of the above embodiment, the included angle ranges from 90° to 170°. In specific applications, the included angle may be set as required. For example, it may be set to 100° or 130° or 150° or the like. For example, in this embodiment, the included angle is set to 120°.

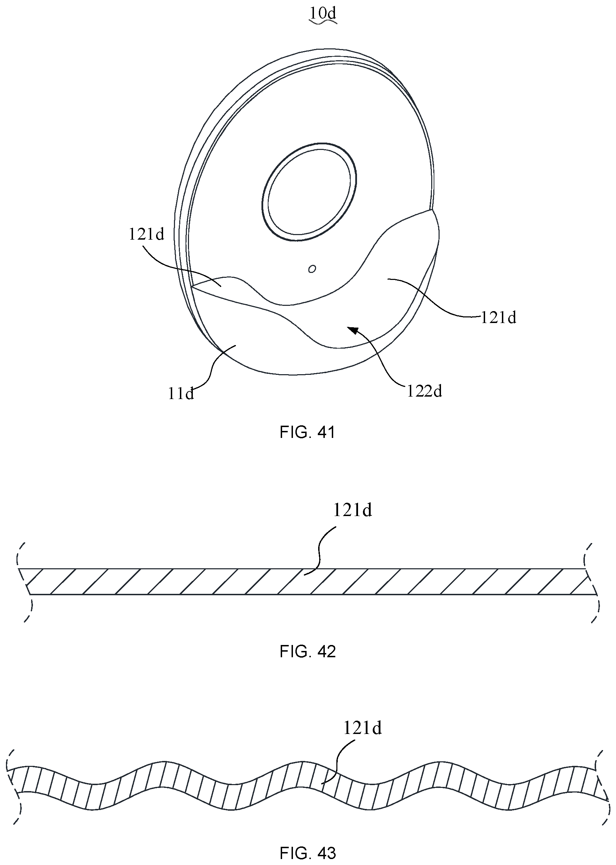

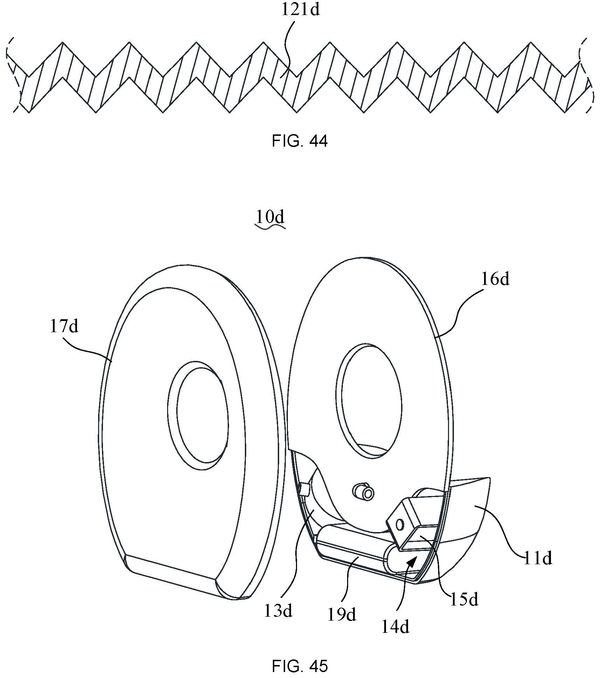

Referring to FIG. 42, each of the panels 121d is planar, which is simple in process, and easy to manufacture. In another embodiment, the panel 121d may also be non-planar. This further increases the surface area of the first top shell 12d to improve the heat transfer efficiency. Referring to FIG. 43 and FIG. 44, the panel 121d may be waved, and may also be in other shapes. The shape of the panel 121d is not limited herein.

As an implementation, two adjacent panels 121d are in smooth transition. This prevents a dead corner between the panels 121d, such that the first accommodating chamber 14d can effectively accommodate components, expanding an accommodation rate of the first accommodating chamber 14d. In addition, the support wall 21d is attached to the first top shell 12d, which also prevents a dead corner of the support wall 21d to facilitate the cleaning of the milk storage container 20d.

As an implementation, referring to FIG. 36 and FIG. 41, the panels 121d form a limiting recess 122d. The limiting recess 122d is configured to limit the milk storage container 20d. In specific applications, the limiting recess 122d is configured to limit a left-right direction of the milk storage container 20d.

As an implementation, the air pump 13d corresponds to a bottom wall of the limiting recess 122d. That is, a position of the first accommodating chamber 14d for accommodating the air pump 13d corresponds to the bottom wall of the limiting recess 122d. The milk flows to a lowest position of the support wall 21d under the action of gravity, and the lowest position of the support wall 21d is attached to the bottom wall of the limiting recess 122d, ensuring that the milk can still effectively dissipate the heat of the air pump 13d, even at an early stage of the milk flowing into the milk storage chamber or when the amount of the milk is small.

As an implementation, one of the first top shell 12d and the support wall 21d is provided with an anti-disengagement snap groove, while the other is provided with an anti-disengagement snap protrusion. The anti-disengagement snap protrusion is snap-fitted into the anti-disengagement snap groove. With snap-fitted assembly between the anti-disengagement snap protrusion and the anti-disengagement snap groove, the connection strength between the first top shell 12d and the support wall 21d can be enhanced, ensuring that the milk storage container 20d is not prone to loosening or even falling off the main unit 10d when being accidentally pulled by an external force.

For example, in this embodiment, a side of the first top shell 12d away from the first accommodating chamber 14d is provided with the anti-disengagement snap protrusion, and a side of the support wall 21d away from the milk storage chamber is provided with the anti-disengagement snap groove.

It is to be noted that, optionally, the anti-disengagement snap protrusion is a spherical protrusion, and the correspondingly matched anti-disengagement snap groove is a spherical groove with a spherical bottom wall. This effectively prevents the milk storage container 20d from moving along a horizontal plane. Alternatively, optionally, the anti-disengagement snap protrusion may be a slide rail structure with a T-shaped cross-section, and the correspondingly matched anti-disengagement snap groove is a slide groove structure with a T-shaped cross-section. In this way, the milk storage container 20d can be slidably disposed on the main unit 10d through the slide groove structure and the slide rail structure, and the milk storage container 20d is limited in an up-down direction.

As an implementation, the main unit 10d further includes a solenoid valve 15d. The air pump 13d and the solenoid valve 15d are accommodated side by side in the first accommodating chamber 14d. The solenoid valve 15d generates heat during operation. By disposing the solenoid valve 15d in the first accommodating chamber 14d, the milk can also serve to dissipate heat of the solenoid valve 15d.

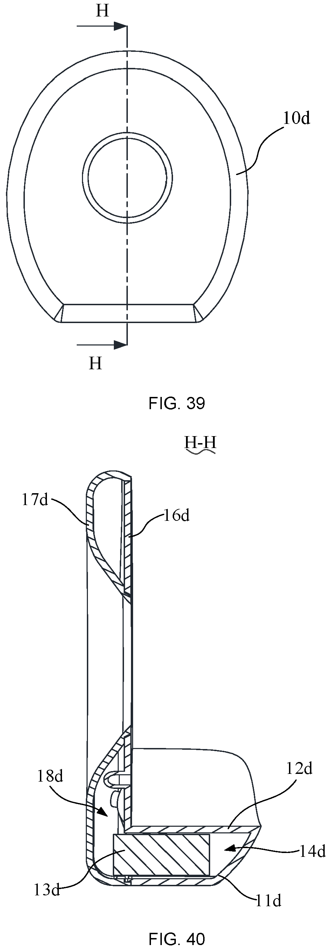

As an implementation, referring to FIG. 40 and FIG. 45, the main unit 10d further includes a second top shell 16d and a second bottom shell 17d. The second top shell 16d and the second bottom shell 17d enclose a second accommodating chamber 18d. The first accommodating chamber 14d communicates with the second accommodating chamber 18d. The main unit 10d further includes a battery 19d and a control panel (not shown in the figure). The battery 19d is accommodated in the first accommodating chamber 14d. The control panel is accommodated in the second accommodating chamber 18d. The battery 19d is electrically connected to the control panel. Both the air pump 13d and the solenoid valve 15d are electrically connected to the control panel.

The battery 19d, the air pump 13d, and the solenoid valve 15d are arranged side by side in the first accommodating chamber 14d. Referring to FIG. 40, FIG. 41, and FIG. 45, the air pump 13d may be disposed between the battery 19d and the solenoid valve 15d, and correspond to the bottom wall of the limiting recess 122d. The battery 19d may also be disposed between the air pump 13d and the solenoid valve 15d, thereby spacing two heat-producing components apart.

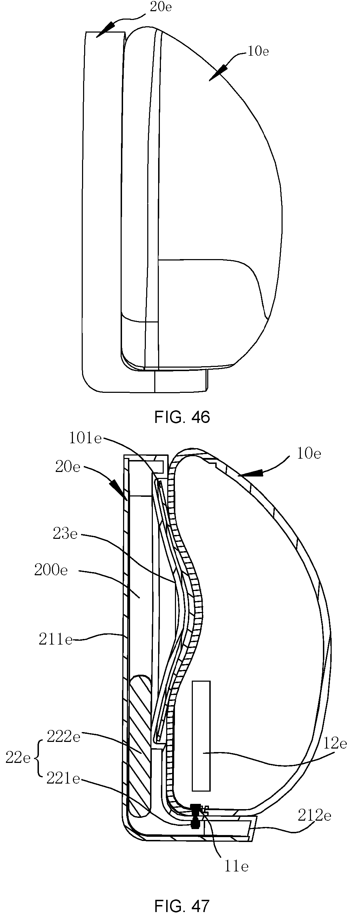

FIG. 46 is a schematic structural view of a breast pump device according to an embodiment of the present disclosure.

Referring to FIG. 46, the breast pump device provided by this embodiment of the present disclosure includes a breast pump 10e and a dust cover 20e having a charging function. The dust cover 20e may be configured to cooperate with the breast pump 10e for use. The breast pump 10e has an opening configured to cover a breast to collect milk. When the breast pump 10e is not used or has low power, the dust cover 20e can shield the opening of the breast pump 10e to protect the opening, preventing entry of dust, bacteria and the like into the breast pump 10e through the opening. Meanwhile, the dust cover 20e can be used to charge the breast pump 10e. When the breast pump 10e needs to be used, it can be used normally by only removing the dust cover 20e. The breast pump 10e mainly includes a main unit and a milk bowl. The milk bowl is configured to store the milk. The main unit is configured to generate a negative pressure during operation, expressing the milk to the milk bowl.

After the dust cover 20e is mounted on the breast pump 10e, the dust cover 20e can shield the opening of the breast pump 10e to prevent entry of dust, bacteria and the like into the breast pump and avoid the contamination and deterioration to the milk in the breast pump 10e. Meanwhile, the breast pump 10e can be charged as required without increasing the size of the breast pump 10e, which balances the milk capacity and the battery endurance of the breast pump 10e, and ensures the portability and user experience of the breast pump 10e.

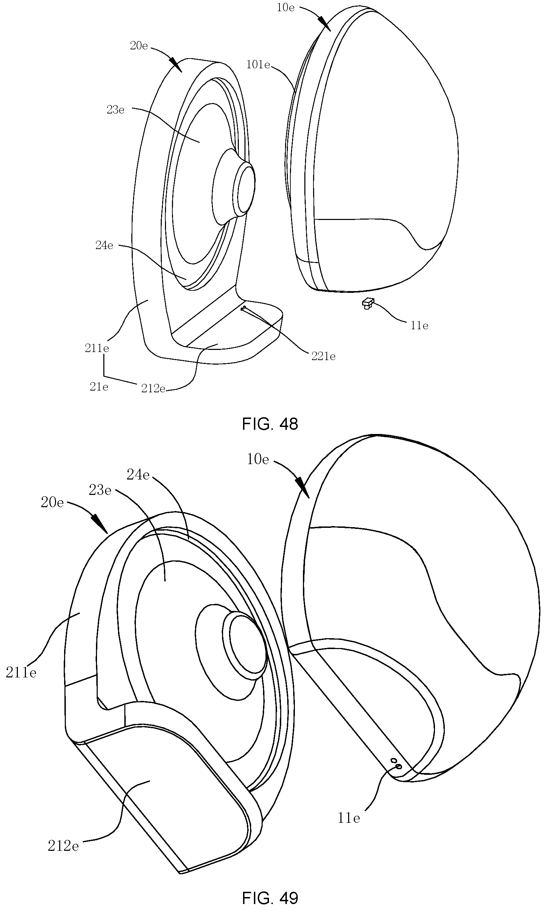

FIG. 47 is a sectional view of a breast pump according to an embodiment of the present disclosure. FIG. 48 is a schematic exploded view of a breast pump device according to an embodiment of the present disclosure. FIG. 49 is another schematic exploded view of a breast pump device according to an embodiment of the present disclosure.

Specifically, as shown in FIGS. 47-49, this embodiment provides the dust cover 20e having a charging function. The dust cover 20e mainly includes a cover body 21e and a charging assembly 22e. The cover body 21e is provided with an accommodating chamber 200e, and configured to be mounted on the breast pump 10e to shield the opening of the breast pump 10e. The charging assembly 22e is at least partially disposed in the accommodating chamber 200e. The charging assembly 22e is configured to charge the breast pump 10e when the cover body 21e is mounted on the breast pump 10e, so as to supplement electric energy to a battery in the breast pump 10e, improving endurance performance of the breast pump 10e.

In an embodiment, a surface of the cover body 21e facing the opening of the breast pump 10e is provided with a protruding conical portion 23e and an accommodating portion 24e recessed at a periphery of the conical portion 23e. The conical portion 23e extends into the opening of the breast pump 10e. The accommodating portion 24e is configured to accommodate at least a portion of the breast pump 10e.

In some embodiments, the accommodating portion 24e may also be omitted.

To achieve better attachment to a human body, a surface of the breast pump 10e facing the human body is provided with a breast shield 101e. The opening is formed in the breast shield 101e. The breast shield 101e is typically made of a soft material, such as silica gel, which can ensure a good attachment effect between the breast shield 101e and the human body. When the breast pump 10e operates, the milk can enter the milk bowl from the opening of the breast shield 101e.

An outer circumferential surface of the conical portion 23e is an inclined truncated conical surface that protrudes outward from a middle, i.e., from the accommodating portion 24e of the dust cover 20e to a direction away from the dust cover 20e. A radial size of the outer circumferential surface of the conical portion 23e gradually decreases. When the dust cover 20e is mounted on the breast pump 10e, the conical portion 23e may cooperate with the opening of the breast pump 10e, thereby guiding the dust cover 20e to be accurately and quickly mounted on the breast pump 10e. After the dust cover 20e is mounted on the breast pump 10e, an end of the breast shield 101e is accommodated in the accommodating portion 24e, and a side wall of the accommodating portion 24e encloses the breast shield 101e therein, achieving a reliable sealing and dust-proof effect.

In an embodiment, the side wall of the accommodating portion 24e may further be provided with a protrusive portion snap-fitted with an edge of the breast shield 101e. For example, the protrusive portion may be a clamping jaw or a limiting edge. The breast shield 101e is limited between a bottom of the accommodating portion 24e and the protrusive portion without disengagement.

Specifically, the breast pump 10e includes a built-in second battery 12e. The second battery 12e is configured to provide electric energy for a main unit of the breast pump 10e. The charging assembly 22e may include an interface module 221e and a power source. The interface module 221e serves as a power output interface, and is configured to match with the breast pump 10e for electrical connection when the cover body 21e is mounted on the breast pump 10e. The power source is electrically connected to the interface module 221e. The power source is a battery or an external power source.

Thus, the dust cover 20e can function as a power bank. Meanwhile, when not used, the dust cover 20e can also be conveniently detached from the breast pump 10e for charging. In addition, the breast pump 10e may also be charged with a built-in charger.

For example, the power source is a first battery 222e. The first battery 222e is connected to the interface module 221e and configured to supply direct current (DC) to the interface module 221e, thereby supplementing electric energy to the second battery 12e in the breast pump 10e through the interface module 221e after the dust cover 20e is mounted on the breast pump 10e. It should be understood that in other embodiments, the charging assembly 22e may also include a power conversion module (not shown in the figure). The power conversion module is connected to the interface module 221e, and is configured to connect an external power source (such as a mains supply or a high-voltage power source), so as to convert an external alternating current (AC) into a DC for subsequent supply to the interface module 221e, or convert an external high voltage into a suitable low-voltage DC for subsequent supply to the interface module 221e. Thus, the dust cover 20e can serve as a charger, eliminating the need for an additional matched charger and reducing the number of components. In another embodiment, the charging assembly 22e may include both the power conversion module and the second battery 12e. The power conversion module and the second battery 12e are connected to the interface module 221e, such that the charging assembly 22e or the external power source can be freely selected by the user as required to charge the breast pump 10e.

Both the power conversion module and the first battery 222e may be disposed in the accommodating chamber 200e. The interface module 221e may be at least partially disposed in the accommodating chamber 200e.

With reference to FIGS. 47-449, this embodiment illustrates a conductive contact manner between the interface module 221e and the breast pump 10e via a conductive terminal. Specifically, the interface module 221e includes a first conductive terminal. The breast pump 10e includes a second conductive terminal 11e. The first conductive terminal is configured to contact the second conductive terminal 11e for electrical conduction when the cover body 21e is mounted on the breast pump 10e.

Specifically, the first conductive terminal is a conductive pin protruding from the cover body 21e. A recess (not shown in the figure) is formed in the breast pump 10e. The second conductive terminal 11e is embedded into the recess. When the housing 21e is mounted on the breast pump 10e, the conductive pin can extend into the recess to contact the second conductive terminal 11e, thereby transmitting electric energy.

In an embodiment, the conductive pin is a pogo pin that may be axially telescopic. In a natural state, the conductive pin is in an extended state and can come into elastic contact with the second conductive terminal 11e, thereby ensuring the reliability of conductive contact between the conductive pin and the second conductive terminal 11e.

Since the second conductive terminal 11e is embedded into the recess without protruding from the breast pump 10e, when the breast pump 10e is held by the user or the breast pump 10e is attached to the human body, the protruding second conductive terminal 11e is not contacted. Therefore, the handfeel in use is not compromised, improving the comfort.

Optionally, the recess is flared and horn-shaped. The first conductive terminal may slide along a surface of the horn-shaped recess to contact the second conductive terminal 11e. Alternatively, an insertion groove configured to allow the first conductive terminal to be inserted into is formed in an end of the second conductive terminal 11e. The first conductive terminal may be inserted into the insertion groove of the second conductive terminal 11e to achieve electrical connection.

It should be understood that in other embodiments, the conductive contact manner between the interface module 221e and the breast pump 10e is not limited thereto. For example, contact points may also be respectively disposed on the interface module 221e and the breast pump 10e. The interface module 221e and the breast pump 10e may come into contact via their respective contact points, thereby realizing the conductive contact between the interface module 221e and the breast pump 10e.

In an embodiment, the cover body 21e is L-shaped, and includes a body portion 211e and a base portion 212e connected to one end of the body portion 211e. The body portion 211e is configured to shield the opening of the breast pump 10e. The base portion 212e is configured to support the breast pump 10e. Exemplarily, the body portion 211e and the connecting body portion 211e are perpendicular to each other. After the dust cover 20e is mounted on the breast pump 10e, the breast pump 10e is entirely supported on the base portion 212e, and the opening of the breast pump 10e faces the body portion 211e and is shielded by the body portion 211e. A bottom of the breast pump 10e may be planar, and a surface of the base portion 212e may also be planar, such that the breast pump 10e can be stably fixed on the surface of the base portion 212e. In other embodiments, the bottom of the breast pump 10e may also be non-planar, and may be an arc surface for example. The surface of the base portion 212e is matched with the bottom of the breast pump 10e in shape. As shown in FIG. 49, for the interface module 221e, the first conductive terminal of the interface module 221e may be disposed on the base portion 212e, i.e., on a top surface of the base portion 212e, while the second conductive terminal 11e is disposed on a bottom of the breast pump 10e. Since the first conductive terminal is not disposed on the body portion 211e, the situation where the second conductive terminal 11e faces the human body to contact the human body is prevented.

It should be understood that in other embodiments, the first conductive terminal of the interface module 221e may also not be disposed on the surface of the base portion 212e facing the bottom of the breast pump 10e. For example, the interface module 221e is disposed on a first surface of the cover body 21e. The first surface is adjacent to a surface of the cover body 21e facing the opening of the breast pump 10e. Exemplarily, the first conductive terminal may be disposed on a side of the body portion 211e or the base portion 212e, so as to extend toward an outer circumferential surface of the breast pump 10e, provided that the second conductive terminal 11e on the breast pump 10e corresponds to the first conductive terminal.

It should be understood that the power transmission manner between the interface module 221e and the breast pump 10e is not limited to conductive contact. Instead, both may also transmit electric energy in a contactless manner. For example, in an embodiment, the interface module 221e includes a first inductive coil. The breast pump 10e includes a second inductive coil. When the cover body 21e is mounted on the breast pump 10e, electric energy can be transmitted between the first inductive coil and the second inductive coil to realize wireless charging. By setting the power transmission manner between the interface module 221e and the breast pump 10e as the contactless manner, the accommodating hole corresponding to the electrical connecting terminal is unnecessarily formed in the surface of the breast pump 10e, and the protrusion of the electrical connecting terminal is not worried. This not only ensures the aesthetic appearance of the product but also improves the handfeel of the product. For example, the first inductive coil may be disposed in the accommodating chamber 200e, and located at a position corresponding to the conical portion 23e, so as to facilitate power transmission with the second inductive coil.

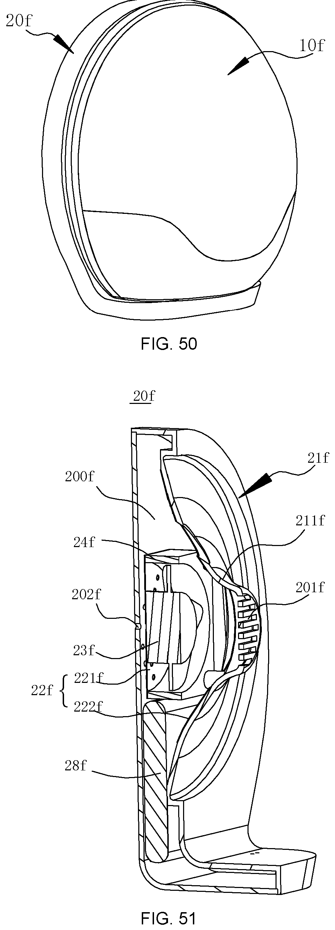

FIG. 50 is a schematic sectional view of a breast pump device according to an embodiment of the present disclosure. FIG. 51 is a schematic sectional view of a dust cover according to an embodiment of the present disclosure.

Referring to FIG. 50 and FIG. 51, the breast pump device provided by this embodiment of the present disclosure includes a breast pump 10f and a dust cover 20f. The dust cover 20f may be configured to cooperate with the breast pump 10f for use. The breast pump 10f has a milk collection opening configured to cover a breast to collect milk. When the breast pump 10f is not used, the dust cover 20f can shield the milk collection opening of the breast pump 10f to protect the milk collection opening, preventing entry of dust, bacteria and the like into the breast pump 10f through the milk collection opening. Meanwhile, the dust cover 20f can be used to preheat the breast pump 10f. When the breast pump 10f needs to be used, it can be used normally by only removing the dust cover 20f.

As shown in FIG. 51, this embodiment of the present disclosure provides the dust cover 20f. The dust cover 20f includes a cover body 21f and a heating assembly 22f. The cover body 21f is configured to be mounted on the breast pump 10f, so as to shield the milk collection opening of the breast pump 10f. A mounting space 200f is formed in the cover body 21f. An air outlet 201f communicating with the mounting space 200f is formed in the cover body 21f. The heating assembly 22f is disposed in the mounting space 200f and configured to heat air in the mounting space 200f. The air heated by the heating assembly 22f in the dust cover 20f may be discharged from the air outlet 201f to preheat the breast pump 10f, preventing discomfort caused by contact between the human body and the excessively cold milk collection opening of the breast pump 10f.

When the breast pump 10f is used, the dust cover 20f is taken down, and then the milk collection opening is attached to the human body. By operating the breast pump 10f, the milk can be expressed from the breast by means of a negative pressure. The milk enters the breast pump 10f through the milk collection opening for temporary storage.

It should be understood that the breast pump 10f may be a manual breast pump, and may also be an electric breast pump. In an embodiment, the breast pump 10f is the electric breast pump, and further includes a main unit. The main unit is configured to generate the negative pressure during operation to express the milk.

The dust cover 20f provided by this embodiment can be mounted on the milk collection opening of the breast pump 10f to protect the breast pump 10f, preventing entry of bacteria, dust and the like into the breast pump 10f. Moreover, due to the heating assembly 22f disposed in the dust cover 20f, when the dust cover 20f is mounted on the breast pump 10f, hot air generated in the dust cover 20f can preheat the breast pump 10f, thereby preventing the cold breast pump 10f from directly contacting the human breast to enhance the user experience.

For example, in winter or cold and damp weather conditions, by mounting the dust cover 20f on the breast pump 10f, and activating the heating assembly 22f, the air in the dust cover 20f can be heated. The hot air is discharged from the air outlet 201f to preheat the breast pump 10f (particularly the milk collection opening). Upon completion of preheating, the milk collection opening is no longer cold, the dust cover 20f is taken down, and the breast pump 10f can be started for use.

Specifically, the dust cover 20f may include a battery 28f. The battery 28f is disposed in the mounting space 200f, electrically connected to the heating assembly 22f, and configured to provide electric energy for the heating assembly 22f. It should be understood that the dust cover 20f may also not include the battery 28f, but use an external power source to supply power to the heating assembly 22f. For example, a power supply interface or a plug may be disposed on the cover body 21f, and power supply can be achieved by connecting the external power source to the power supply interface or the plug.

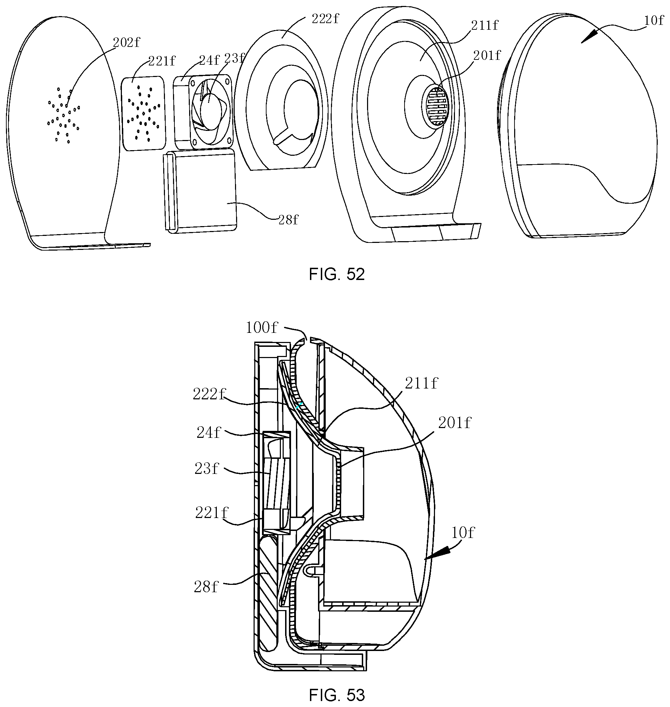

As shown in FIG. 51 and FIG. 52, in an embodiment, besides the air outlet 201f, the cover body 21f further includes an air inlet 202f. The air inlet 202f and the air outlet 201f communicate with the mounting space 200f. Outside air can enter the mounting space 200f through the air inlet 202f, and flow out through the air outlet 201f after being heated by the heating assembly 22f. To accelerate discharge of the hot air from the mounting space 200f, the dust cover 20f further includes a convection fan 23f. The convection fan 23f is disposed in the mounting space 200f and configured to draw the outside air into the mounting space 200f from the air inlet 202f, and blow the air heated by the heating assembly 22f out from the air outlet 201f. In this way, the outside air can be drawn in rapidly, and the heated air can be discharged rapidly at the same time.

In an embodiment, the heating assembly 22f includes a first heating element 221f. The first heating element 221f is disposed between the air inlet 202f and the convection fan 23f. That is, the first heating element 221f is located on an upstream side of the convection fan 23f. The outside air entering from the air inlet 202f is heated by the first heating element 221f, and then drawn by the convection fan 23f toward the air outlet 201f. In this process, the air entering the convection fan 23f is hot air.

In an embodiment, the heating assembly 22f may include a second heating element 222f. The second heating element 222f is disposed between the convection fan 23f and the air outlet 201f. That is, the second heating element 222f is located on a downstream side of the convection fan 23f. The outside air entering from the air inlet 202f is drawn in by the convection fan 23f and then provided for the second heating element 222f. The hot air heated by the second heating element 222f is blown out by the convection fan 23f toward the air outlet 201f. In this process, the air entering the convection fan 23f is outside air at a room temperature.

It should be understood that in another embodiment, the heating assembly 22f may include at least two heating elements. For example, the heating assembly 22f includes the first heating element 221f and the second heating element 222f. The first heating element 221f is disposed between the air inlet 202f and the convection fan 23f. The second heating element 222f is disposed between the convection fan 23f and the air outlet 201f. Under the action of the convection fan 23f, the outside air is drawn in from the air inlet 202f, heated by the first heating element 221f, and blown out by the convection fan 23f toward the second heating element 222f. Hot air heated by the first heating element 221f is reheated by the second heating element 222f, and reheated hot air is blown out from the air outlet 201f under the action of the convection fan 23f.

In an embodiment, the heating assembly 22f includes at least one heating element. The at least one heating element is a heating sheet, and/or the at least one heating element is a heating wire, and/or the at least one heating element is a heating mesh. That is, both the first heating element 221f and the second heating element 222f may be selected from the heating sheet, the heating wire, and the heating mesh. The first heating element 221f and the second heating element 222f may be a same type of heating elements, and may also be different types of heating elements. The heating assembly 22f may further include more than two heating elements.

As shown in FIG. 51 and FIG. 52, the first heating element 221f is the heating wire, and the second heating element 222f is the heating sheet. The first heating element 221f, the convection fan 23f, and the second heating element 222f are sequentially disposed in the mounting space 200f along an airflow direction. The second heating element 222f is an annular heating sheet, with an airflow channel formed in a middle thereof communicating with the air outlet 201f. The air blown by the convection fan 23f toward the second heating element 222f is heated by the second heating element 222f, and flows out from the air outlet 201f through the airflow channel in the middle of the second heating element 222f.

Specifically, the dust cover 20f further includes a cylindrical guide shell 24f mounted in the mounting space 200f. The convection fan 23f is disposed in the guide shell 24f. Two ends of the guide shell 24f face the air inlet 202f and the air outlet 201f, respectively. Exemplarily, an extension direction of a central axis of the guide shell 24f is the same as the airflow direction of the convection fan 23f. The guide shell 24f may be configured to guide air to enter the convection fan 23f, and guide the air to flow out from the convection fan 23f. The first heating element 221f may be fixed on an upstream end of the guide shell 24f.

Further with reference to FIG. 53, a side of the cover body 21f facing the milk collection opening is convexly provided with a conical guide portion 211f. The air outlet 201f is formed in an end surface of a free end of the guide portion 211f. The air inlet 202f is formed in a side of the cover body 21f facing away from the milk collection opening. After the dust cover 20f is mounted on the breast pump 10f, the air may enter the mounting space 200f from the side of the cover body 21f facing away from the milk collection opening. Since the end surface of the free end of the guide portion 211f directly faces the milk collection opening of the breast pump 10f, the hot air flowing out from the dust cover 20f may directly flow to the milk collection opening. There may be more than one air outlet 201f. For example, a plurality of air outlets 201f are arranged in an array in the end surface of the free end of the guide portion 211f.

It should be understood that the air outlet 201f may also be formed in the outer circumferential surface of the guide portion 211f in whole or in part. For example, a portion of the air outlet 201f is formed in the end surface of the free end of the guide portion 211f, while another portion of the air outlet 201f is formed in the outer circumferential surface of the guide portion 211f. In an embodiment, the air inlet 202f may also be formed in the outer circumferential surface of the cover body 21f.

In an embodiment, when the dust cover 20f is configured to be mounted on the milk collection opening of the breast pump 10f, a gap is formed between the dust cover 20f and the milk collection opening. Therefore, heated air discharged from the air outlet 201f can be discharged from at least one of a milk pouring opening 100f of the breast pump 10f and the gap. For example, a portion of the hot air is discharged from the gap between the dust cover 20f and the milk collection opening after preheating the milk collection opening, and can also blow away dust, dirt and the like at the gap to clean the milk collection opening of the breast pump 10f. Another portion of the hot air can enter the milk bowl of the breast pump 10f through the milk collection opening and then flow out through the milk pouring opening 100f.

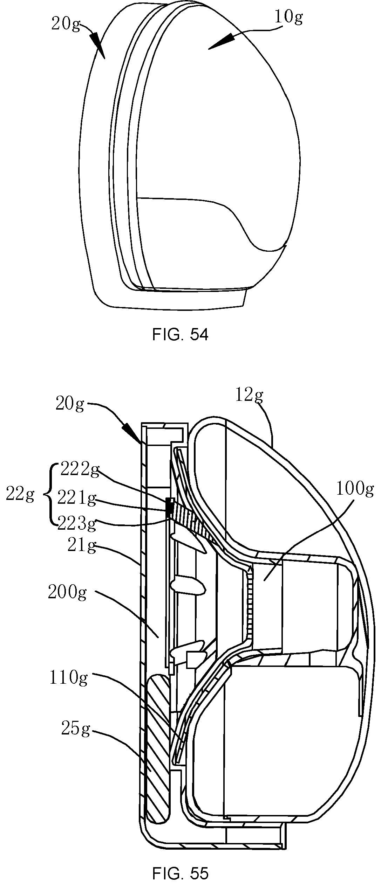

FIG. 54 is a schematic structural view of a breast pump device according to an embodiment of the present disclosure. FIG. 55 is a sectional view of a breast pump according to an embodiment of the present disclosure.

Referring to FIG. 54 and FIG. 55, the breast pump device provided by this embodiment of the present disclosure includes a breast pump 10g and a dust cover 20g. The dust cover 20g may be configured to cooperate with the breast pump 10g for use, and detachably connected to the breast pump 10g. The breast pump 10g has a milk collection opening 100g configured to cover a breast to collect milk. When the breast pump 10g is not used, the dust cover 20g can shield the milk collection opening 100g of the breast pump 10g to protect the milk collection opening 100g, preventing entry of dust, bacteria and the like into the breast pump 10g through the milk collection opening 100g. Meanwhile, the dust cover 20g can be used to disinfect and sterilize the breast pump 10g. When the breast pump 10g needs to be used, it can be used normally by only removing the dust cover 20g.

The breast pump 10g mainly includes a flange 110g and a milk bowl 12g. The milk bowl 12g is mounted on the flange 110g, and configured to store milk. The flange 110g has the milk collection opening 100g. A milk collection channel is formed in the milk bowl 12g. The milk collection opening 100g communicates with an interior of the milk bowl through the milk collection channel. It should be understood that a breast shield may be integrally or detachably disposed on the flange 110g. The milk collection opening 100g is formed in the breast shield.

When the breast pump is used, the dust cover 20g is taken down, and then the milk collection opening 100g is attached to the human body. By operating the breast pump 10g, the milk can be expressed from the breast by means of a negative pressure. The milk enters the milk bowl 12g through the milk collection opening 100g for temporary storage.

It should be understood that the breast pump 10g may be a manual breast pump, and may also be an electric breast pump. In an embodiment, the breast pump 10g is the electric breast pump, and further includes a main unit. The main unit is configured to generate the negative pressure during operation to express the milk to the milk bowl 12g.

After the dust cover 20g is mounted on the breast pump 10g, the dust cover 20g can shield the opening of the breast pump 10g to prevent entry of dust, bacteria and the like into the breast pump and avoid the contamination and deterioration to the milk in the breast pump 10g. Meanwhile, the milk collection opening 100g of the breast pump 10g can also be disinfected as required to prevent contamination to the milk in the breast pump 10g due to bacterial reproduction at the milk collection channel, ensuring the health of infants.

As shown in FIG. 55, this embodiment provides the dust cover 20g. The dust cover 20g includes a cover body 21g and a disinfection assembly 22g. The cover body 21g is configured to be mounted on the breast pump 10g, so as to shield the milk collection opening 100g of the breast pump 10g. A mounting space 200g is formed in the cover body 21g. The cover body 21g has a light exiting side facing the milk collection opening 100g. The disinfection assembly 22g is at least partially disposed in mounting space 200g and configured to emit ultraviolet light through the light exiting side to disinfect the milk collection opening 100g of the breast pump 10g.

Since the ultraviolet disinfection function is integrated onto the dust cover 20g rather than the breast pump 10g, the size of the breast pump 10g is not increased, and the dust cover 20g is fully utilized, realizing versatility. The dust cover 20g not only has dust-proof and antibacterial functions, but also can disinfect the breast pump 10g during idle time when the breast pump 10g is not used. When disinfection is required, only the disinfection assembly 22g is activated to irradiate the ultraviolet light onto the milk collection opening 100g.

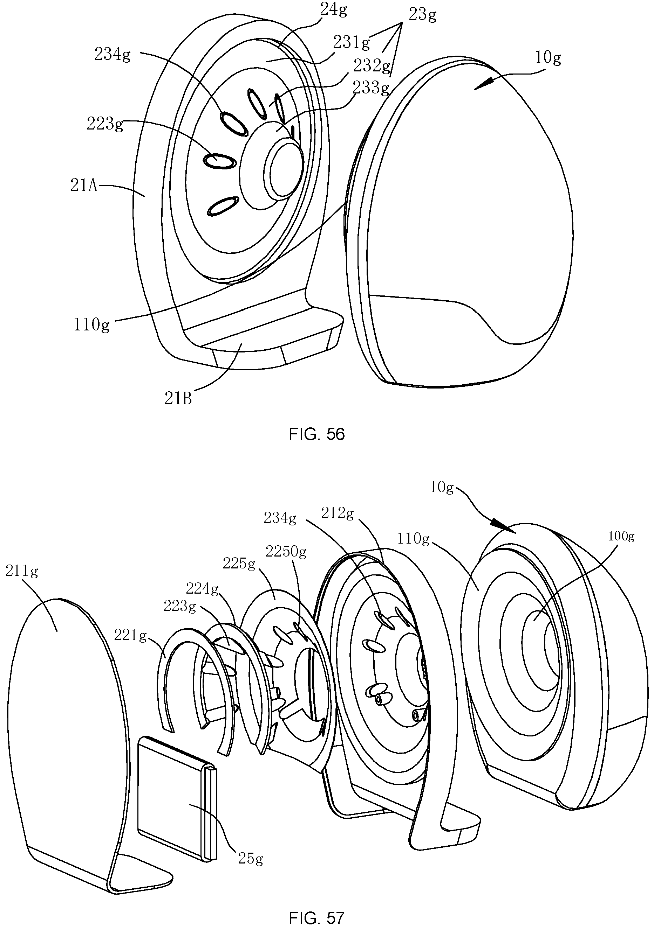

FIG. 56 is another schematic exploded view of a breast pump device according to an embodiment of the present disclosure.

Further with reference to FIG. 56, in an embodiment, the light exiting side of the cover body 21g is provided with a conical protrusive portion 23g and an accommodating portion 24g around the protrusive portion 23g. The accommodating portion 24g is configured to allow the breast pump 10g to be partially embedded into when the protrusive portion 23g enters the milk collection opening 100g. Specifically, a circumferential edge of the breast shield is embedded into the accommodating portion 24g. The protrusive portion 23g is accommodated in the breast shield. The conical protrusive portion 23g is configured to be gradually small toward a free end. When the dust cover 20g is mounted on the breast pump 10g, the conical protrusive portion 23g slides along a conical surface of the breast shield, and can guide the dust cover 20g to shield the milk collection opening 100g of the breast pump 10g.

In an embodiment, the milk bowl 12g is at least partially made of a transparent material. For example, a side of the milk bowl 12g facing away from the flange 110g is transparent, such that the user can view an internal structure of the breast pump 10g from the outside. This novel design also enables the user to easily observe an amount of milk stored in the milk bowl 12g, thereby enhancing the practicability of the breast pump 10g. Specifically, the milk collection opening 100g is formed in the flange 110g, and the breast shield is made of an opaque material. For example, the flange 110g is entirely made of the opaque material, such that the ultraviolet light emitted by the disinfection assembly 22g is irradiated onto the opaque milk collection opening 100g, rather than the milk bowl 12g, preventing the ultraviolet light from entering the human eyes to cause harm to the user.

By designing an exiting angle of the ultraviolet light of the disinfection assembly 22g, the ultraviolet light can only be irradiated onto the opaque milk collection opening 100g. Exemplarily, the protrusive portion 23g includes an annular conical surface 232g. The ultraviolet light emitted by the disinfection assembly 22g exits through the conical surface 232g, and obliquely exits to the milk collection opening 100g. In an embodiment, the conical surface 232g is entirely light-transmissive. The ultraviolet light emitted by the disinfection assembly 22g exits through the conical surface 232g. In another embodiment, a plurality of light-transmitting windows 234g are formed in the conical surface 232g. The ultraviolet light emitted by the disinfection assembly 22g only exits through a corresponding light-transmitting window 234g, such that the ultraviolet light can be limited to exit according to a preset path. It should be understood that the light-transmitting window 234g may be a through hole, and may also be a channel made of a light-transmitting material and configured to allow the ultraviolet light to pass through.

Exemplarily, besides the conical surface 232g, the protrusive portion 23g may further include a first surface 231g and a second surface 233g that are respectively connected to two ends of the conical surface 232. Both the first surface 231g and the second surface 233g are annular. The first surface 231g is located near one side of the accommodating portion 24g, and the second surface 233g is located on the free end. After the dust cover 20g is mounted on the breast pump 10g, the second surface 233g is close to the milk bowl, and the first surface 231g is close to an edge of the milk collection opening 100g. Both the first surface 231g and the second surface 233g may be conical surfaces, and may also be planes.

Specifically, in this embodiment, the disinfection assembly 22g includes a lamp panel 221g and a plurality of ultraviolet light sources 222g that are disposed in the mounting space 200g and electrically connected. The lamp panel 221g is fixed on the cover body 21g. The ultraviolet light sources 222g are disposed on the lamp panel 221g at intervals along a circumferential direction of the cover body 21g and face the light exiting side.

Exemplarily, the milk collection opening 100g includes a breast accommodating chamber and a nipple channel. The breast accommodating chamber is closer to the human body than the nipple channel. The nipple channel communicates with the breast accommodating chamber and the milk bowl 12g. The disinfection assembly 22g includes at least one ultraviolet light source 222g. A light exiting direction of the ultraviolet light source 22g is configured to incline toward a center of the nipple channel. In this way, the ultraviolet light source 22g can be irradiated onto a region corresponding to the nipple channel of the milk collection opening 100g.

In an embodiment, as shown in FIG. 56 and FIG. 57, the cover body 21g may include a rear cover 211g and a front cover 212g. The rear cover 211g and the front cover 212g cooperate to form the mounting space 200g. The lamp panel 221g is fixed on the rear cover 211g. The ultraviolet light source 222g is fixed on the lamp panel 221g. The protrusive portion 23g and the accommodating portion 24g are formed on the front cover 212g. For example, the cover body 21g may be L-shaped, and both the rear cover 211g and the front cover 212g are L-shaped. The cover body 21g includes a body portion 21A configured to cover the milk collection opening 100g and a base portion 21B connected to a bottom of the body portion 21A. In this case, the protrusive portion 23g and the accommodating portion 24g are formed on the body portion 21A. Exemplarily, the body portion 21A and the base portion 21B may be perpendicular to each other. When the body portion 21A covers the milk collection opening 100g of the breast pump 10g, the base portion 21B can support the breast pump 10g, such that the breast pump 10g can be stably placed on a plane.

In an embodiment, the lamp panel 221g is annular or C-shaped. The ultraviolet light sources 222g are distributed at intervals in a circumferential direction of the lamp panel 221g. Therefore, the light emitted by each ultraviolet light source 222g is converged to a corresponding region of the milk collection opening 100g to disinfect different portions, thereby ensuring the disinfection effect.

In addition, to enhance the light-guiding effect, in an embodiment, the disinfection assembly 22g may include a plurality of light guide posts 223g. Each of the light guide posts 223g includes one end facing the ultraviolet light sources 222g, and another end extending to the conical surface 232g, and inclining toward a center of the conical surface 232g, so as to guide the ultraviolet light emitted by the ultraviolet light sources 222g to exit through the conical surface 232g.

Since the free end of the light guide post 223g inclines, the light emitted by the ultraviolet light sources 222g is projected at a certain angle to the milk collection opening 100g of the breast pump 10g, ensuring that the ultraviolet light is irradiated onto the milk collection opening 100g rather than the milk bowl 12g, thus achieving a certain light-guiding effect.

Accordingly, when the light-transmitting window 234g is the through hole, the light guide post 223g extends out of the light-transmitting window 234g to guide the ultraviolet light to a specific region of the breast pump 10g. When the light-transmitting window 234g is the light-transmitting material rather than the through hole, the light guide post 223g may abut against or may be aligned at the light-transmitting window 234g.

Further, the disinfection assembly 22g may further include a light guide ring 224g. The light guide post 223g is connected to the light guide ring 224g. A surface of the light guide ring 224g facing away from the light guide post 223g is attached to a surface of the lamp panel 221g provided with the ultraviolet light sources 222g. Exemplarily, the light guide ring 224g and the light guide post 223g is integrally formed, which may facilitate assembly and improve the assembly efficiency.

In addition, the disinfection assembly 22g may further include a light homogenizing sheet 225g. The light homogenizing sheet 225g is attached to an inner surface of the protrusive portion 23g and directly faces the conical surface 232g. The light emitted by the ultraviolet light source 222g can be irradiated onto the light homogenizing sheet 225g. After undergoing homogenization treatment by the light homogenizing sheet 225g, the light features better uniformity. Moreover, the light homogenizing sheet 225g can collect the scattered light and reuse it for a second time, thereby improving the light utilization rate. When the disinfection assembly 22g includes the light guide post 223g, a plurality of limiting holes 2250g may further be formed in the light homogenizing sheet 225g. The light guide post 223g can pass through a corresponding limiting hole 2250g, and the limiting hole 2250g can limit the light guide post 223g to some extent.

In an embodiment, the conical surface 232g may also be a reflective surface. The light exiting from the light-transmitting window 234g can be reflected by the conical surface 232g and then further converged to the breast pump 10g, enhancing the light intensity.

In addition, in an embodiment, the dust cover 10g further includes a battery 25g disposed in the mounting space 200g. The battery 25g is electrically connected to the disinfection assembly 22g to supply power to the disinfection assembly 22g. Therefore, the dust cover 10g can disinfect the breast pump 20g without the external power source. This can be suitable for more portable scenarios. It should be understood that in other embodiments, the dust cover 10g may also not include the battery, but the external power source is required to supply power to the disinfection assembly 22g.

The foregoing is merely an alternative example of the present application and does not constitute a limitation on the scope of the present patent application. Any equivalent structure or equivalent process change made under the application concept of the present application by using the description and the accompanying drawings of the present application, or direct or indirect application thereof in other related technical fields, shall still fall in the protection scope of the patent of this application.

## Claims

1. A breast pump assembly, configured for a breast pump, and comprising:
a container body and a sealing cover, wherein the container body and the sealing cover enclose a milk storage chamber;
a negative pressure member, wherein the negative pressure member is connected to the sealing cover, the negative pressure member has a negative pressure chamber, and the negative pressure chamber is configured to communicate with a negative pressure hole of the breast pump; and
a milk guide member, wherein the milk guide member is connected to the sealing cover, the milk guide member has a milk guide channel, the milk guide channel communicates with the negative pressure chamber, and the milk guide channel is at least configured to guide milk from a breast;
wherein the negative pressure member, the milk guide member, and the sealing cover are integrally formed, and one of the milk guide channel and the negative pressure chamber communicates with the milk storage chamber.

2. The breast pump assembly according to claim 1, wherein both the milk guide member and the negative pressure member are connected to a side of the sealing cover facing the milk storage chamber.

3. The breast pump assembly according to claim 2, wherein a first perforation and a second perforation are formed in the sealing cover; and a central axis of the first perforation is parallel to a central axis of the second perforation;
the negative pressure member is connected to the first perforation; and the first perforation is configured to communicate the negative pressure chamber with the negative pressure hole; and
the milk guide member is connected to the second perforation; and the second perforation communicates with the milk guide channel, and is configured to allow the milk to pass through and flow to the milk guide channel.

4. The breast pump assembly according to claim 3, wherein the central axis of the first perforation is coaxial with a central axis of the negative pressure chamber; and the central axis of the second perforation is coaxial with a central axis of the milk guide channel.

5. The breast pump assembly according to claim 3 or 4, wherein the first perforation is closely adjacent to the second perforation; and/or,
when the breast pump is placed against the breast, the first perforation is located under the second perforation.

6. The breast pump assembly according to claim 5, wherein when the breast pump is placed against the breast, the first perforation is located directly under the second perforation, and the negative pressure chamber communicates with the milk storage chamber.

7. The breast pump assembly according to any one of claims 1 to 4, wherein the container body is provided with a first mounting groove; and the sealing cover is accommodated in the first mounting groove, so as to enclose the milk storage chamber with the container body.

8. A breast pump, comprising:
a breast shield, wherein the breast shield is provided with a breast accommodating chamber;
a main unit, wherein the main unit is provided with a negative pressure hole configured to transfer a negative pressure; and
the breast pump assembly according to any one of claims 1 to 7, wherein the milk guide channel communicates with the breast accommodating chamber; and the negative pressure chamber communicates with the negative pressure hole.

9. The breast pump according to claim 8, wherein the first perforation is formed in the sealing cover;
and the negative pressure member is connected to the first perforation and located on the side of the sealing cover facing the milk storage chamber;
the breast pump further comprises an elastic diaphragm; and the elastic diaphragm comprises a diaphragm body and a turnup connected to the diaphragm body; and
a circumferential direction of the main unit around the negative pressure hole and/or a circumferential direction of the sealing cover around the first perforation is provided with a second mounting groove; the turnup is disposed in the second mounting groove; and the diaphragm body is accommodated in the negative pressure chamber.

10. The breast pump according to claim 9, wherein the negative pressure member further has a first through hole and a second through hole; the first through hole is configured to communicate the negative pressure chamber with the milk guide channel; the second through hole is configured to communicate the negative pressure chamber with the milk storage chamber; and when the breast pump is placed against the breast, the negative pressure chamber is located under the milk guide channel; and
the elastic diaphragm has a contracted state and an expanded state; and when the elastic diaphragm is in the expanded state, at least a portion of the elastic diaphragm corresponding to the first through hole is spaced apart from a wall of the negative pressure chamber.

11. A breast pump, comprising:
a milk guide member, wherein the milk guide member is made of an optically transparent material and has a milk guide channel, and the milk guide channel is at least configured to guide milk from a breast;
a negative pressure member, wherein the negative pressure member has a negative pressure chamber, and the negative pressure chamber at least communicates with the milk guide channel; and
a main unit, wherein the main unit is provided with a negative pressure hole, and the negative pressure hole communicates with the negative pressure chamber;
wherein when the breast pump is placed against the breast, the negative pressure member is located under the milk guide member, and the main unit is located at a position other than on the milk guide member.

12. The breast pump according to claim 11, wherein at least a portion of the main unit is disposed on a side of the negative pressure member away from the milk guide member.

13. The breast pump according to claim 11 or 12, wherein the negative pressure member is made of the optically transparent material.

14. The breast pump according to claim 11 or 12, wherein the breast pump further comprises a milk storage container; both the milk guide member and the negative pressure member are connected to an inner side of the milk storage container; and the main unit is disposed on an outer side of the milk storage container; and
when the breast pump is placed against the breast, at least a portion of the milk storage container located above the milk guide member is made of the optically transparent material.

15. The breast pump according to claim 14, wherein the milk storage container is entirely made of the optically transparent material.

16. The breast pump according to claim 15, wherein the milk storage container comprises a first container wall and a second container wall; and the first container wall and the second container wall enclose a milk storage chamber;
both the negative pressure member and the milk guide member are connected to a side of the second container wall facing the milk storage chamber; and when the breast pump is placed against the breast, the first container wall is located above the milk guide member; and
transparency of the first container wall is greater than or equal to transparency of the second container wall.

17. The breast pump according to claim 11 or 12, wherein the breast pump further comprises a breast shield; and
the breast shield comprises a breast attachment portion and a channel portion connected to the breast attachment portion; the breast attachment portion is configured to be adsorbed to the breast; the channel portion is provided with a nipple channel; and at least a portion of the channel portion extends into the milk guide member, such that the nipple channel communicates with the milk guide channel.

18. The breast pump according to claim 17, wherein the channel portion is made of the optically transparent material.

19. The breast pump according to claim 17, wherein the channel portion is in an interference fit with the milk guide member; or,
an outer surface of the channel portion is smooth; or,
the breast shield is detachably connected to the milk guide member.

20. The breast pump according to claim 17, wherein an end of the channel portion away from the breast attachment portion and an inner surface of the milk guide member define a step, so as to prevent backflow of the milk.

21. A breast pump assembly, configured for a breast pump, and comprising a negative pressure member, wherein the negative pressure member has a negative pressure chamber, and the negative pressure chamber is configured to communicate with a negative pressure hole of the breast pump; and
a milk guide member, wherein the milk guide member has a milk guide channel, the milk guide channel communicates with the negative pressure chamber, and the milk guide channel is at least configured to guide milk from a breast;
wherein the milk guide member and the negative pressure member are arranged side by side in the breast pump.

22. The breast pump assembly according to claim 21, wherein when the breast pump is placed against the breast, the negative pressure member is located under the milk guide member.

23. The breast pump assembly according to claim 21, wherein the milk guide member and the negative pressure member share a first side wall; and
two opposite sides of the first side wall face the negative pressure chamber and the milk guide channel, respectively; and a first through hole configured to communicate the negative pressure chamber with the milk guide channel is formed in the first side wall.

24. The breast pump assembly according to claim 23, wherein when the breast pump is placed against the breast, the first through hole is located at a lowest position of the milk guide member.

25. The breast pump assembly according to claim 23, wherein the negative pressure member further comprises a second side wall and a first bottom wall; and the first side wall, the second side wall, and the first bottom wall enclose the negative pressure chamber; and
a second through hole configured to communicate with the negative pressure chamber is formed in the second side wall; and when the breast pump is placed against the breast, the second through hole is located under the first through hole.

26. The breast pump assembly according to claim 25, wherein when the breast pump is placed against the breast, the second through hole is located at a lowest position of the negative pressure member.

27. The breast pump assembly according to claim 25, wherein when the breast pump is placed against the breast, the second through hole directly faces the first through hole along a vertical direction; or,
the second through hole is formed in an end of the second side wall close to the first bottom wall.

28. The breast pump assembly according to claim 21, wherein a length of the milk guide member is the same as a length of the negative pressure member; and/or,
an opening of the negative pressure chamber for communicating with the negative pressure hole and an opening of the milk guide channel for receiving the milk are arranged side by side and have a same orientation.

29. The breast pump assembly according to any one of claims 21 to 28, wherein the breast pump assembly comprises a container body and a sealing cover; the container body and the sealing cover enclose a milk storage chamber; and the milk guide member and the negative pressure member are connected side by side to a same surface of the sealing cover and extend toward one side of the milk storage chamber.

30. A breast pump, comprising:
a breast shield, wherein a breast accommodating chamber is formed in the breast shield;
a main unit, wherein the main unit is provided with a negative pressure hole configured to transfer a negative pressure; and
the breast pump assembly according to any one of claims 21 to 29, wherein the milk guide channel communicates with the breast accommodating chamber, and the negative pressure chamber communicates with the negative pressure hole.

31. A breast pump, comprising:
a main unit housing, wherein the main unit housing comprises a first shell and a second shell extending relative to the first shell in a bending manner, so as to form a step on the main unit housing; and an accommodating chamber is formed in the main unit housing;
a milk pumping assembly, wherein the milk pumping assembly is at least partially located on the step; and
a pumping power device that is disposed in the accommodating chamber, wherein the pumping power device is configured to generate a negative pressure.

32. The breast pump according to claim 31, wherein the milk pumping assembly comprises a milk collection unit and a breast shield; the milk collection unit is disposed on one side of the first shell; and the second shell is disposed between the breast shield and the milk collection unit; and
at least one of the first shell and the second shell has a cavity, so as to form the accommodating chamber.

33. The breast pump according to claim 32, wherein the first shell has a first cavity; and the second shell has a second cavity;
the accommodating chamber comprises the first cavity and the second cavity; and
at least a portion of the pumping power device is accommodated in the first cavity.

34. The breast pump according to claim 33, wherein the pumping power device comprises an air pump, a solenoid valve, a battery, and a circuit board; and
the air pump, the solenoid valve, and the battery are accommodated in the first cavity; and the circuit board is accommodated in the second cavity.

35. The breast pump according to claim 34, wherein the air pump, the battery, and the solenoid valve are arranged side by side; and the battery is located between the air pump and the solenoid valve.

36. The breast pump according to claim 33, wherein a negative pressure hole is formed in a side of the second shell provided with the first shell; and the negative pressure hole communicates with the second cavity.

37. The breast pump according to claim 32, wherein the breast pump further comprises a key unit; and the key unit is disposed on a circumferential side, other than an end close to the first shell, of the second shell.

38. The breast pump according to claim 32, wherein the second shell is further provided with a through hole; the through hole extends from a side of the second shell away from the first shell to a side of the second shell close to the first shell; and the through hole is configured to allow the breast shield to penetrate through.

39. The breast pump according to claim 32, wherein the milk collection unit is supported on the first shell; and/or,
the main unit housing is L-shaped; and an L-shaped step is formed on the main unit housing.

40. The breast pump according to any one of claims 31 to 39, wherein the milk collection unit comprises a milk storage container and a breast pump assembly extending into the milk storage container; and
the pumping power device is configured to provide the negative pressure for the breast pump assembly.

41. A breast pump, comprising:
a main unit, wherein the main unit is configured to provide a negative pressure; and
a milk storage container, wherein the milk storage container comprises a support wall and an enclosure wall, the support wall and the enclosure wall enclose a milk storage chamber, and
the support wall is configured to support milk flowing to the milk storage chamber;
wherein at least a portion of the main unit is attached to a side of the support wall away from the milk storage chamber.

42. The breast pump according to claim 41, wherein the main unit comprises a first bottom shell, a first top shell, and an air pump;
the first bottom shell and the first top shell enclose a first accommodating chamber; and the air pump is accommodated in the first accommodating chamber; and
the first top shell is attached to the side of the support wall away from the milk storage chamber.

43. The breast pump according to claim 42, wherein a projected contour of the first top shell along a direction toward the support wall falls within a contour of the support wall.

44. The breast pump according to claim 42, wherein a thickness of the first top shell is less than or equal to a thickness of the first bottom shell.

45. The breast pump according to claim 42, wherein the first top shell comprises at least two panels;
and any two adjacent ones of the panels are disposed at an included angle; and
the included angle is less than 180°.

46. The breast pump according to claim 45, wherein each of the panels is non-planar; and/or, two adjacent ones of the panels are in smooth transition.

47. The breast pump according to claim 45, wherein the at least two panels define a limiting recess; and the limiting recess is configured to limit the milk storage container.

48. The breast pump according to claim 47, wherein the air pump corresponds to a bottom wall of the limiting recess.

49. The breast pump according to claim 42, wherein one of the first top shell and the support wall is provided with an anti-disengagement snap groove, while the other is provided with an anti-disengagement snap protrusion; and the anti-disengagement snap protrusion is snap-fitted into the anti-disengagement snap groove.

50. The breast pump according to any one of claims 42 to 49, wherein the main unit further comprises a solenoid valve; and
the air pump and the solenoid valve are accommodated side by side in the first accommodating chamber.

51. A dust cover having a charging function, configured to cooperate with a breast pump for use, and comprising:
a cover body, wherein an accommodating chamber is formed in the cover body; and the cover body is configured to be mounted on the breast pump, so as to shield an opening of the breast pump; and
a charging assembly that is at least partially disposed in the accommodating chamber, and
configured to charge the breast pump when the cover body is mounted on the breast pump.

52. The dust cover having a charging function according to claim 51, wherein the charging assembly comprises:
an interface module that is configured to match with the breast pump for electrical connection when the cover body is mounted on the breast pump; and
a power source that is electrically connected to the interface module, wherein the power source is a battery or an external power source.

53. The dust cover having a charging function according to claim 52, wherein the interface module comprises a first conductive terminal; the breast pump comprises a second conductive terminal; and the first conductive terminal is configured to contact the second conductive terminal for electrical conduction when the cover body is mounted on the breast pump.

54. The dust cover having a charging function according to claim 53, wherein the first conductive terminal is a conductive pin protruding from the cover body; a recess is formed in the breast pump; the second conductive terminal is embedded into the recess; and when the cover body is mounted on the breast pump, the conductive pin extends into the recess to contact the second conductive terminal.

55. The dust cover having a charging function according to claim 52, wherein the interface module comprises a first inductive coil; the breast pump comprises a second inductive coil; and when the cover body is mounted on the breast pump, electric energy is transmitted between the first inductive coil and the second inductive coil.

56. The dust cover having a charging function according to claim 55, wherein a surface of the cover body facing the opening of the breast pump is provided with a protruding conical portion; and the first inductive coil is disposed in the accommodating chamber, and located at a position corresponding to the conical portion.

57. The dust cover having a charging function according to claim 53, wherein the cover body is L-shaped; the cover body comprises a body portion and a base portion connected to the body portion in a bending manner; the body portion is configured to cover the opening of the breast pump; and the base portion is configured to support the breast pump.

58. The dust cover having a charging function according to claim 57, wherein the first conductive terminal is disposed on a top surface of the base portion.

59. The dust cover having a charging function according to any one of claims 52 to 57, wherein the interface module is disposed on a first surface of the cover body; and the first surface is adjacent to the surface of the cover body facing the opening of the breast pump.

60. A breast pump device, comprising: a breast pump and the dust cover having a charging function according to any one of claims 51 to 59.

61. A dust cover, configured to cooperate with a breast pump for use, and comprising:
a cover body, wherein the cover body is configured to be mounted on the breast pump, so as to shield a milk collection opening of the breast pump, a mounting space is formed in the cover body, and an air outlet communicating with the mounting space is formed in the cover body; and
a heating assembly, wherein the heating assembly is disposed in the mounting space and configured to heat air in the mounting space, and the air heated by the heating assembly is discharged from the air outlet to preheat the breast pump.

62. The dust cover according to claim 61, wherein the cover body further comprises an air inlet communicating with the mounting space; and the dust cover further comprises:
a convection fan that is disposed in the mounting space and configured to draw outside air into the mounting space from the air inlet, and blow the air heated by the heating assembly out from the air outlet.

63. The dust cover according to claim 62, wherein the heating assembly comprises a first heating element; and the first heating element is disposed between the air inlet and the convection fan.

64. The dust cover according to claim 62, wherein the heating assembly comprises a second heating element; and the second heating element is disposed between the convection fan and the air outlet.

65. The dust cover according to claim 62, wherein the heating assembly comprises at least one heating element; and the at least one heating element is a heating sheet, and/or the at least one heating element is a heating wire, and/or the at least one heating element is a heating mesh.

66. The dust cover according to claim 62, wherein the dust cover further comprises a cylindrical guide shell mounted in the mounting space; the convection fan is disposed in the guide shell; and two ends of the guide shell face the air inlet and the air outlet, respectively.

67. The dust cover according to any one of claims 61 to 66, wherein a side of the cover body facing the milk collection opening is convexly provided with a conical guide portion; and the air outlet is formed in an end surface and/or an outer circumferential surface of a free end of the guide portion.

68. The dust cover according to any one of claims 62 to 66, wherein the air inlet is formed in a side of the cover body facing away from the milk collection opening and/or an outer circumferential surface of the cover body.

69. A breast pump device, comprising: a breast pump and the dust cover according to any one of claims 61 to 68.

70. The breast pump device according to claim 69, wherein when the dust cover is configured to be mounted on the milk collection opening of the breast pump, a gap is formed between the dust cover and the milk collection opening; and heated air discharged from the air outlet is discharged from at least one of a milk pouring opening of the breast pump and the gap.

71. A dust cover, configured to cooperate with a breast pump for use, and comprising:
a cover body, wherein the cover body is configured to be mounted on the breast pump, so as to shield a milk collection opening of the breast pump, a mounting space is formed in the cover body, and the cover body has a light exiting side facing the milk collection opening; and
a disinfection assembly that is at least partially disposed in the mounting space and configured to emit ultraviolet light through the light exiting side to disinfect the milk collection opening of the breast pump.

72. The dust cover according to claim 71, wherein the light exiting side of the cover body is provided with a conical protrusive portion and an accommodating portion around the protrusive portion; the accommodating portion is configured to allow the breast pump to be partially embedded into when the protrusive portion enters the milk collection opening; and the protrusive portion comprises an annular conical surface; and
the conical surface is light-transmissive, or, a plurality of light-transmitting windows are formed in the conical surface.

73. The dust cover according to claim 72, wherein the disinfection assembly comprises a lamp panel and a plurality of ultraviolet light sources that are disposed in the mounting space and electrically connected; the lamp panel is fixed on the cover body; and the ultraviolet light sources are disposed on the lamp panel at intervals along a circumferential direction of the cover body and face the light exiting side.

74. The dust cover according to claim 73, wherein the disinfection assembly comprises a plurality of light guide posts; and each of the light guide posts comprises one end facing the ultraviolet light sources, and another end extending to the conical surface, and inclining toward a center of the conical surface, so as to guide the ultraviolet light emitted by the ultraviolet light sources to exit through the conical surface.

75. The dust cover according to claim 74, wherein the disinfection assembly comprises a light guide ring; the light guide post is connected to the light guide ring; and a surface of the light guide ring facing away from the light guide post is attached to a surface of the lamp panel provided with the ultraviolet light sources.

76. The dust cover according to claim 72, wherein the disinfection assembly comprises a light homogenizing sheet; and the light homogenizing sheet is attached to an inner surface of the protrusive portion and directly faces the conical surface.

77. The dust cover according to claim 72, wherein the conical surface is a reflective surface.

78. The dust cover according to claim 71, wherein the milk collection opening comprises a breast accommodating chamber and a nipple channel; the disinfection assembly comprises at least one ultraviolet light source; and a light exiting direction of the ultraviolet light source is configured to incline toward a center of the nipple channel.

79. A breast pump device, comprising: a breast pump and the dust cover according to any one of claims 71 to 78, wherein the dust cover is detachably connected to the breast pump.

80. The breast pump device according to claim 79, wherein the breast pump comprises a flange and a milk bowl disposed on the flange; the milk bowl is at least partially made of a transparent material; a breast shield having the milk collection opening is formed on the flange; the breast shield is made of an opaque material; and the ultraviolet light emitted by the disinfection assembly is irradiated onto the milk collection opening.
